(19) Europäisches Patentamt
European Patent Office
Office européen des brevets

(11) Numéro de publication: **0 428 442 B1**

(12) # FASCICULE DE BREVET EUROPEEN

(45) Date de publication de fascicule du brevet: **31.08.94**

(51) Int. Cl.⁵: **A61K 7/13**, C07D 209/08

(21) Numéro de dépôt: **90403178.8**

(22) Date de dépôt: **08.11.90**

Le dossier contient des informations techniques présentées postérieurement au dépôt de la demande et ne figurant pas dans le présent fascicule.

(54) **Compositions et procédés de teinture mettant en oeuvre comme coupleurs des dérivés de 6- ou 7-hydroxyindole.**

(30) Priorité: **10.11.89 FR 8914795**

(43) Date de publication de la demande:
**22.05.91 Bulletin 91/21**

(45) Mention de la délivrance du brevet:
**31.08.94 Bulletin 94/35**

(84) Etats contractants désignés:
**AT BE CH DE DK ES FR GB GR IT LI NL SE**

(56) Documents cités:
**EP-A- 0 416 962**
**FR-A- 2 626 771**
**GB-A- 2 207 443**
**GB-A- 2 211 517**

**S.T.N. SERVEUR DE BASES DE DONNEES, Karlsruhe, DE, FICHIER REGISTRY;& RN=22132-17-8**

(73) Titulaire: **L'OREAL**
**14, Rue Royale**
**F-75008 Paris (FR)**

(72) Inventeur: **Junino, Alex**
**16, rue Docteur Bergonié**
**F-93180 Livry-Gargan (FR)**
Inventeur: **Vandenbossche, Jean-Jacques**
**37, Avenue Berlioz,**
**F-93270 Sevran (FR)**
Inventeur: **Richard, Hervé**
**48, rue de l'Ermitage**
**F-75020 Paris (FR)**
Inventeur: **Cotteret, Jean**
**15, Allée des Meuniers**
**F-78480 Verneuil-sur-Seine (FR)**

(74) Mandataire: **Casalonga, Axel et al**
**BUREAU D.A. CASALONGA - JOSSE**
**Morassistrasse 8**
**D-80469 München (DE)**

EP 0 428 442 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à de nouvelles compositions tinctoriales pour fibres kératiniques et en particulier pour cheveux humains, contenant des précurseurs de colorants d'oxydation et des coupleurs dérivés de 6- ou 7-hydroxyindole, et un procédé de teinture mettant en oeuvre de telles compositions.

Il est connu de teindre les fibres kératiniques et en particulier les cheveux humains, avec des compositions tinctoriales contenant des précurseurs de colorants d'oxydation et en particulier des p-phénylènediamines, des ortho- ou para-aminophénols appelés généralement "bases d'oxydation".

On sait également qu'on peut faire varier les nuances obtenues avec ces bases d'oxydation en les associant à des coupleurs encore appelés modificateurs de coloration, choisis notamment parmi des métadiamines aromatiques, des méta-aminophénols et des métadiphénols.

On recherche, dans le domaine de la teinture capillaire, des précurseurs de colorants d'oxydation ou des coupleurs permettant de conférer aux cheveux, en milieu alcalin oxydant généralement utilisé en teinture d'oxydation, une coloration ayant une résistance satisfaisante à la lumière, aux lavages, aux intempéries et à la transpiration.

La demanderesse vient de découvrir, ce qui fait l'objet de l'invention, qu'un procédé mettant en oeuvre certains dérivés de 6- ou 7-hydroxyindole à titre de coupleurs, avec des précurseurs de colorants d'oxydation, permettait d'obtenir après application sur les fibres kératiniques et en particulier les cheveux, des teintures présentant des résistances à la lumière, aux lavages, aux intempéries et à la transpiration, particulièrement remarquables, notamment lorsqu'ils sont utilisés avec la p-phénylènediamine et ses dérivés. Un objet de l'invention est donc constitué par cette utilisation.

L'invention a également pour objet des compositions tinctoriales d'oxydation, destinées à être utilisées pour la teinture des fibres kératiniques, contenant au moins un précurseur de colorant d'oxydation de type para et/ou ortho avec certains dérivés indoliques définis ci-après.

Un autre objet de l'invention est constitué par le procédé de coloration des fibres kératiniques, en particulier des chevaux humains, mettant en oeuvre de telles compostions.

D'autres objets de l'invention apparaîtront à la lecture de la description et des exemples qui suivent.

Les composés dérivés du 6- ou 7-hydroxyindole utilisés comme coupleurs dans la teinture d'oxydation des fibres kératiniques et en particulier des cheveux humains, en présence d'au moins un précurseur de colorant d'oxydation para et/ou ortho répondent à la formule :

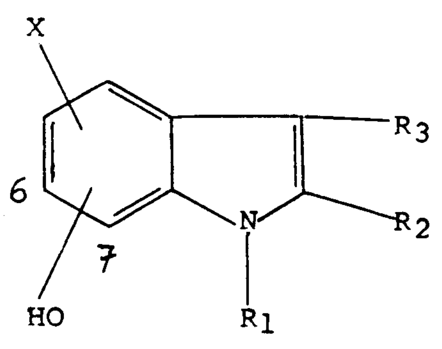

(I)

dans laquelle $R_1$ désigne un atome d'hydrogène, un radical alkyle en $C_1$-$C_4$; $R_2$ et $R_3$, identiques ou différents, désignent un atome d'hydrogène, un radical alkyle inférieur en $C_1$-$C_4$, un radical carboxyle ou un radical alcoxycarbonyle; X désigne un radical alkyle inférieur en $C_1$-$C_4$, un radical alcoxy en $C_1$-$C_{18}$, un atome d'halogène, un radical acyloxy en $C_1$-$C_{20}$, un groupement acétylamino, un groupement triméthylsilyloxy, ou un groupement dialkyl ($C_1$-$C_4$) aminométhyl; le groupement OH occupant la position 6 ou 7 du cycle aromatique; ainsi que leurs sels.

Parmi les composés de formule (I), les composés préférés sont les composés dans lesquels le radical alkyle désigne méthyle, éthyle; le radical alcoxycarbonyle désigne méthoxy ou éthoxycarbonyle; le radical alcoxy désigne méthoxy, éthoxy, butoxy, hexadécyloxy; le radical acyloxy désigne acétoxy, tétradécanoyloxy.

Parmi ces composés, on peut citer le 6-hydroxy 5-acétoxyindole, le 6-hydroxy 5-méthoxyindole, le 6-hydroxy 5-méthoxy 2-méthylindole, le 6-hydroxy 5-butoxyindole, l'acide 6-hydroxy 5-méthoxyindole 2-carboxylique, le 6-hydroxy 7-méthoxyindole, le 6-hydroxy 5-méthoxy 2,3-diméthylindolel le 7-hydroxy 4-méthoxy 2,3-diméthylindole, le 6-hydroxy 5-tétradécanoyloxyindole, le 6-hydroxy 5-hexadécyloxyindole, le

6-hydroxy 5-butanoyloxyindole, le 6-hydroxy 5-oléyloxy indole, le 6-hydroxy 5-éthanoyloxyindole, le 6-hydroxy 5-hexanoyloxyindole, le 6-hydroxy 5-pivaloyloxyindole, le 6-hydroxy 5-formyloxyindole, le 6-hydroxy 5-triméthyl silyloxyindole, le 6-hydroxy 7-méthylindole, le 7-hydroxy 6-diméthylaminométhylindole, le 7-hydroxy 6-méthoxy 2-méthylindole, le 6-hydroxy 7-méthoxy 2-éthoxy carbonylindole, le 6-hydroxy 7-diméthylamino méthylindole, le 6-hydroxy 3,7-diméthylindole, le 6-hydroxy 3-méthyl 7-diméthylaminométhylindole, le 6-hydroxy 5-méthoxy 1-méthylindole, le 6-hydroxy 5-méthyl -2-carboxylindole, le 6-hydroxy 7-méthoxy 2-méthylindole, le 6-hydroxy 5-méthoxy 2-méthylindole, le 6-hydroxy 5-acétylamino 2,3-diméthylindole.

Le 6-hydroxy 5-acétylamino 2,3-diméthylindole est un composé nouveau et constitue un autre objet de l'invention.

Les précurseurs de colorants de type para ou ortho sont des composés qui ne sont pas des colorants en eux-mêmes, mais qui forment un colorant par un processus de condensation oxydative, soit sur eux-mêmes, soit en présence d'un coupleur ou modificateur.

Ces composés comportent des groupements fonctionnels, soit deux amino, soit un amino et un hydroxy, en position para ou ortho, l'un par rapport à l'autre.

Les précurseurs de type para sont en particulier choisis parmi les paraphénylènediamines, les para-aminophénols, les précurseurs hétérocycliques para, comme la 2,5-diaminopyridine, la 2-hydroxy 5-aminopyridine, la tétraaminopyrimidine et les bases dites "doubles".

A titre de paraphénylènediamines, on peut citer les composés répondant à la formule (II) ci-après :

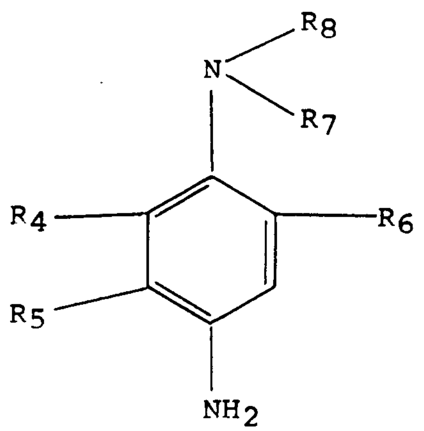

(II)

dans laquelle $R_4$, $R_5$ et $R_6$, identiques ou différents, représentent un atome d'hydrogène ou d'halogène, un radical alkyle ayant 1 à 4 atomes de carbone, un radical alcoxy ayant 1 à 4 atomes de carbone, $R_7$ et $R_8$, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, pipéridinoalkyle, morpholinoalkyle, ces groupes alkyle ou alcoxy ayant de 1 à 4 atomes de carbone, ou bien $R_7$ et $R_8$ forment, conjointement avec l'atome d'azote auquel ils sont liés, un hétérocycle pipéridino ou morpholino, sous réserve que $R_4$ ou $R_6$ représente un atome d'hydrogène lorsque $R_7$ et $R_8$ ne représentent pas un atome d'hydrogène, ainsi que les sels de ces composés.

Parmi les composés de formule (II), on peut citer la p-phénylènediamine, la p-toluylènediamine, la méthoxyparaphénylènediamine, la chloroparaphénylènediamine, la 2,6-diméthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di($\beta$-hydroxyéthyl)paraphénylènediamine, la 3-méthyl 4-amino N,N-di-($\beta$-hydroxyéthyl)aniline, la 3-chloro 4-amino N,N-di-($\beta$-hydroxyéthyl)aniline, la 4-amino N,N-(éthyl, carbamylméthyl)aniline, la 3-méthyl 4-amino N,N-(éthyl, carbamylméthyl)aniline, la 4-amino N,N-(éthyl,$\beta$-pipéri dinoéthyl)aniline, la 3-méthyl 4-amino N,N-(éthyl,$\beta$-pipéridinoéthyl)aniline, la 4-amino N,N-(éthyl,$\beta$-morpholinoéthyl)aniline, la 3-méthyl 4-amino N,N-(éthyl,$\beta$-morpholinoéthyl)aniline, la 4-amino N,N-(éthyl,$\beta$-acétylaminoéthyl)aniline, la 4-amino N-($\beta$-méthoxyéthyl) aniline, la 3-méthyl 4-amino N,N-(éthyl,$\beta$-acétylamino éthyl)aniline, la 4-amino N,N-(éthyl,$\beta$-mésylaminoéthyl) aniline, la 3-méthyl 4-amino N,N-(éthyl,$\beta$-mésylaminoéthyl)aniline, la 4-amino N,N-(éthyl,$\beta$-sulfoéthyl) aniline, la 3-méthyl 4-amino N,N-(éthyl,$\beta$-sulfoéthyl) aniline, la N-[(4'-amino)phényl]-morpholine, la N-[(4'-amino)phényl]pipéridine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 2,5-diméthyl 4-aminophénol, le 2-méthoxyméthyl 4-aminophénol.

Les colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène, les orthophénylènediamines.

Les bases dites doubles sont des bis-phénylalkylènediamines, répondant à la formule :

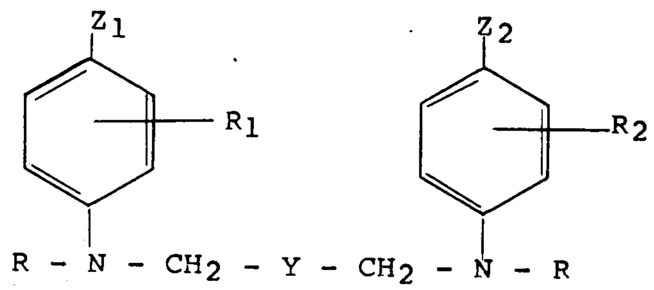

(III)

dans laquelle :

$Z_1$ et $Z_2$, identiques ou différents, représentent des groupements hydroxyle ou $NHR_3$, où $R_3$ désigne un atome d'hydrogène ou un radical alcoyle inférieur;

$R_1$ et $R_2$, identiques ou différents, représentent soit des atomes d'hydrogène, soit des atomes d'halogène, soit encore des groupements alcoyle;

R représente un atome d'hydrogène, un groupe alcoyle, hydroxyalcoyle ou aminoalcoyle, dont le reste amino peut être substitué;

Y représente un radical pris dans le groupe constitué par les radicaux suivants : $-(CH_2)_n-$, $(CH_2)_n$, $-O-(CH_2)_{n'}-$, $-(CH_2)_{n'}-CHOH-(CH_2)_{n'}-(CH_2)_{n'}$,

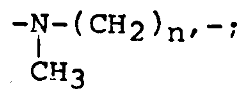

n étant un nombre entier compris entre 0 et 8 et n' un nombre entier compris entre 0 et 4, cette base pouvant se présenter sous forme de ses sels d'addition avec des acides.

Les radicaux alkyle ou alcoxy désignent de préférence un groupement ayant 1 à 4 atomes de carbone et notamment méthyle, éthyle, propyle, méthoxy, éthoxy.

Parmi les composés de formule (III), on peut citer le N,N'bis-(β-hydroxyéthyl)N,N'-bis(4'-amino phényl)-1,3-diamino 2-propanol, la N,N'bis-(β-hydroxyéthyl)N,N'-bis(4'-aminophényl)éthylènediamine, la N,N' bis-(4-aminophényl)tétraméthylènediamine, la N,N'bis-(β-hydroxyéthyl )N,N'bis(4-aminophényl)tétraméthylène diamine, la N,N'bis-(4-méthylaminophényl)tétraméthylène diamine, la N,N'bis(éthyl)N,N'-bis(4'-amino 3'-méthylphényl)éthylènediamine.

On peut éventuellement utiliser également en plus du coupleur hétérocyclique de la famille des 6-ou 7-hydroxyindoles de formule (I) définie ci-dessus, d'autres coupleurs connus en eux-mêmes, tels que les métadiphénols, les métaamino-phénols, les métaphénylènediamines, les métaacylamino-phénols, les métauréidophénols, les métacarbalcoxyaminophénols, 1' α-naphtol, les coupleurs possédant un groupement méthylène actif, tels que les composés β-cétoniques, les pyrazolones ou le 4-hydroxyindole.

Parmi ces coupleurs, on peut plus particulièrement citer le 2,4-dihydroxyphénoxyéthanol, le 2,4-dihydroxyanisole, le métaaminophénol, le monométhyléther de résorcine, le 2-méthyl 5-N-(β-hydroxyéthyl)-aminophénol, le 2-méthyl 5-N-(β-mésylamino éthyl)aminophénol, la 6-hydroxybenzomorpholine, le 2,4-diaminoanisole, le 2,4-diaminophénoxyéthanol, la 6-aminobenzomorpholine, le [2-N-(β-hydroxyéthyl)amino 4-amino]-phénoxyéthanol, le 2-amino 4-N-(β-hydroxyéthyl)amino anisole, le (2,4-diamino)phényl-β,γ-dihydroxypropyléther, la 2,4-diaminophénoxyéthylamine, le 1,3-diméthoxy 2,4-diaminobenzène, le 2-méthyl 5-aminophénol, le 2,6-diméthyl 3-aminophénol et leurs sels.

On peut utiliser conjointement avec les composés précités et comme cela est bien connu dans l'état de la technique, notamment en vue de nuancer ou d'enrichir en reflets les colorations apportées par les précurseurs de colorants d'oxydation et le coupleur de formule (I), des colorants directs, tels que des colorants azoïques, anthraquinoniques ou les dérivés nitrés de la série benzénique.

On n'utilise pas conjointement aux coupleurs de formule (I) de dérivé quinonique de la famille des benzoquinones ou des naphtoquinones, susceptible d'oxyder le composé de formule (I) ni d'ion iodure.

Les composés précités sont mis en oeuvre à l'aide de compositions tinctoriales d'oxydation contenant dans un milieu approprié pour la teinture au moins un précurseur de colorant d'oxydation para et/ou orho et au moins à titre de coupleur le dérivé de 6- ou 7-hydroxyindole de formule (I) défini ci-dessus, ainsi qu'éventuellement les autres coupleurs et/ou autres colorants directs.

Ces compositions ne doivent pas contenir d'ion iodure dans des proportions susceptibles d'oxyder le précurseur et le coupleur de formule (I).

Les compositions tinctoriales qui constituent un autre objet de l'invention sont essentiellement caractérisées par le fait qu'elles contiennent dans un milieu approprié pour la teinture :

a) au moins un coupleur de formule (I)

b) au moins un précurseur de colorant d'oxydation para et/ou ortho choisi parmi les paraaminophénols, les précurseurs hétérocycliques, les orthoaminophénols, les orthophénylènediamines, les bases dites "doubles" telles que définies ci-dessus, les paraphénylènediamines de formule (IV) :

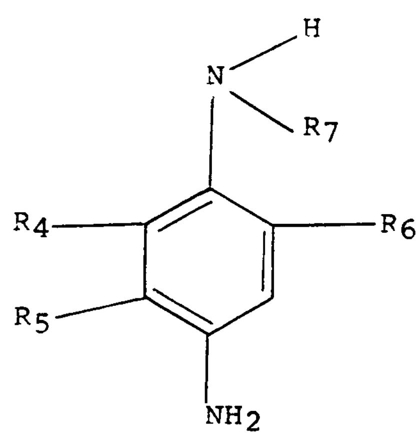

(IV)

dans laquelle $R_4$, $R_5$ et $R_6$, identiques ou différents, représentent un atome d'hydrogène ou d'halogène, un radical alkyle ayant 1 à 4 atomes de carbone, un radical alcoxy ayant 1 à 4 atomes de carbone, $R_7$ représente un atome d'hydrogène, un radical alkyle, carbamylalkyle, mésylaminoalkyle, acétylaminoalkyle, uréidoalkyle, carbalcoxyaminoalkyle, pipéridino-alkyle, morpholino alkyle, ces groupes alkyle ou alcoxy ayant de 1 à 4 atomes de carbone, ainsi que les sels de ces composés.

Parmi les composés de formule (IV), on peut citer la p-phénylènediamine, la p-toluylènediamine, la méthoxyparaphénylènediamine, la chloroparaphénylènediamine, la 2,6-diméthylparaphénylènediamine, la 2,5-diméthylparaphénylènediamine, la 2-méthyl 5-méthoxy paraphénylènediamine, la 2,6-diméthyl 5-méthoxypara phénylènediamine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Ces compositions ne contiennent pas d'ion iodure dans des quantités susceptibles d'oxyder les précurseurs et coupleurs en présence.

L'ensemble des précurseurs de colorants par oxydation de type para et/ou ortho, ainsi que les coupleurs utilisés dans les compositions tinctoriales conformes à l'invention, représente de préférence de 0,3 à 7% en poids par rapport au poids de ladite composition. La concentration en composés (I) peut varier entre 0,05 et 3,5% en poids par rapport au poids total de la composition.

Le milieu approprié pour la teinture est constitué généralement par un milieu aqueux et son pH peut varier entre 8 et 11, et il est de préférence compris entre 9 et 11.

Il est ajusté à la valeur désirée à l'aide d'un agent alcalinisant, tel que l'ammoniaque, les carbonates alcalins, les alcanolamines, comme la mono-, la di- ou la triéthanolamine.

Les compositions tinctoriales conformes à l'invention contiennent également, dans leur forme de réalisation préférée, des agents tensio-actifs anioniques, cationiques, non-ioniques, amphotères ou leurs mélanges. Parmi ces agents tensio-actifs, on peut citer les alkylbenzènesulfonates, les alkylnaphtalènesulfonates, les sulfates, les éthersulfates et les sulfonates d'alcools gras, les sels d'ammonium quaternaires, tels que le bromure de triméthylcétylammonium, le bromure de cétylpyridinium; les éthanolamides d'acides gras éventuellement oxyéthylénés; les acides, les alcools ou les amines polyoxyéthylénés, les alcools polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0,5 et 55% en poids, et de préférence entre 2 et 50% en poids par rapport au poids total de la composition.

Ces compositions peuvent également contenir des solvants organiques pour solubiliser les composés qui ne seraient pas suffisamment solubles dans l'eau. Parmi ces solvants, on peut citer à titre d'exemple, les alcanols inférieurs en $C_1$-$C_4$, tels que l'éthanol et l'isopropanol; le glycérol; les glycols ou éthers de glycols, comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol et les produits analogues ou leurs mélanges.

Les solvants sont présents de préférence dans des proportions comprises entre 1 et 40% en poids, et en particulier entre 5 et 30% en poids par rapport au poids total de la composition.

Les agents épaississants que l'on peut ajouter dans les compositions conformes à l'invention peuvent être choisis parmi l'alginate de sodium, la gomme arabique, les dérivés de cellulose, les polymères d'acide acrylique, la gomme de xanthane. On peut également utiliser des agents épaississants minéraux, tels que la bentonite.

Ces agents épaississants sont présents de préférence dans des proportions comprises entre 0,1 et 5%, et en particulier entre 0,2 et 3% en poids par rapport au poids total de la composition.

Les agents antioxydants qui peuvent être présents dans les compositions sont choisis en particulier parmi le sulfite de sodium, l'acide thioglycolique, le bisulfite de sodium, l'acide ascorbique, l'hydroquinone et l'acide homogentisique. Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquides, de crèmes, de gels ou sous toute autre forme appropriée pour réaliser une teinture des fibres kératiniques et notamment des cheveux humains. Ces compositions peuvent être conditionnées en flacons aérosols en présence d'un agent propulseur.

Le procédé de teinture des fibres kératiniques et en particulier des cheveux humains, conforme à l'invention met en oeuvre la révélation par le peroxyde d'hydrogène.

Conformément à ce procédé, on applique sur les fibres kératiniques une composition préparée au moment de l'emploi, contenant au moins un coupleur de formule (I), au moins un précurseur de colorant d'oxydation para et/ou ortho, et au moins une solution oxydante à base de peroxyde d'hydrogène en une quantité suffisante pour pouvoir développer une coloration.

On utilise de préférence une solution d'eau oxygénée à 20 volumes. Le mélange obtenu est appliqué sur les cheveux et on laisse poser pendant 10 à 40 minutes, de préférence 15 à 30 minutes, après quoi on rince les cheveux, on les lave au shampooing, on les rince à nouveau et on les sèche.

L'agent oxydant peut être introduit tout juste savant l'application de la composition appliquée dans le deuxième temps ou bien être additionné sur les fibres kératiniques, dans un troisième temps, les conditions de pose et de séchage ou de lavage étant similaires à celles indiquées ci-dessus.

Les exemples qui suivent sont destinés à illustrer l'invention.

On prépare la composition suivante :

## COMPOSITION A

| | | |
|---|---|---|
| . Colorants | | x g |
| . Octyldodécanol vendu sous la dénomination d'EUTANOL G par la Société HENKEL | | 8,0 g |
| . Acide oléique | | 20,0 g |
| . Lauryl éther sulfate de monoéthanolamine vendu sous la dénomination de SIPON LM 35 par la Société HENKEL | | 3,0 g |
| . Alcool éthylique | | 10,0 g |
| . Alcool benzylique | | 10,0 g |
| . Alcool cétylstéarylique à 33 moles d'oxyde d'éthylène vendu sous la dénomination de SIMULSOL GS par la Société SEPPIC | | 2,4 g |
| . Acide éthylène diamine tétracétique | | 0,2 g |
| . Polymère cationique constitué de motifs récurrents : 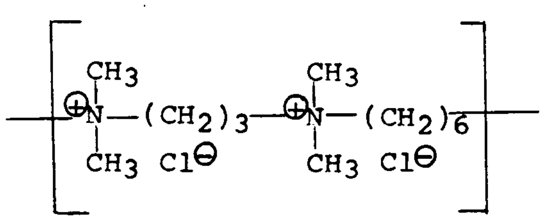 | | 2,2 g |
| . Monoéthanolamine | | 7,5 g |
| . Diéthanolamide d'acide linoléique vendu sous la dénomination de COMPERLAN F par la Société HENKEL | | 8,0 g |
| . Ammoniaque à 20% de NH 3 | | 10,2 g |
| . Métabisulfite de sodium en solution aqueuse à 35% | | 1,3 g |
| . Hydroquinone | | 0,15 g |
| . 1-phényl 3-méthyl 5-pyrazolone | | 0,20 g |
| . Eau déminéralisée | qsp | 100,0 g |

Dans tous les exemples qui suivent, on introduit les colorants dans les quantités indiquées dans le tableau. On mélange la composition contenant le colorant poids pour poids avec un agent oxydant titrant à 20 volumes en eau oxygénée et dont le pH est de 3.

Les mélanges ainsi réalisés sont appliqués 30 minutes sur des cheveux gris à 90% de blancs, puis rincés, lavés, rincés à nouveau et ensuite séchés.

TABLEAU I

| Exemples | Coupleur hétérocyclique de formule (I) | g | Précurseur para | g | Couleur |
|---|---|---|---|---|---|
| 1 | 2,3-diméthyl 5-méthoxy 6-hydroxyindole | 0,382 | p-phénylènediamine | 0,216 | blond clair cendré |
| 2 | 5-méthoxy 6-hydroxyindole | 0,652 | p-aminophénol | 0,436 | blond cuivré cendré |
| 3 | 7-méthoxy 6-hydroxyindole | 0,326 | p-phénylènediamine | 0,216 | blond cuivré doré cendré |
| 4 | 7-méthoxy 6-hydroxyindole | 0,652 | p-aminophénol | 0,436 | blond cuivré doré |
| 5 | 5-tétradécanoyloxy 6-hydroxyindole | 1,438 | p-aminophénol | 0,436 | blond foncé marron chaud |
| 6 | 5-acétoxy 6-hydroxyindole | 0,765 | p-aminophénol | 0,436 | blond foncé marron chaud |
| 7 | 2-méthyl 5-méthoxy 6-hydroxyindole | 0,354 | p-phénylènediamine | 0,216 | blond clair beige |
| 8 | 2,3-diméthyl 4-méthoxy 7-hydroxyindole | 0,382 | p-phénylènediamine | 0,216 | blond beige nacré |

TABLEAU II

| Exemples | Coupleur hétérocyclique de formule (I) | g | Précurseur para | g | Couleur |
|---|---|---|---|---|---|
| 9 | 2,3-diméthyl 4-méthoxy 7-hydroxyindole | 0,764 | p-aminophénol | 0,436 | blond cuivré beige |
| 10 | 6-hydroxy 5-butanoyloxy indole | 0,438 | p-phénylènediamine | 0,216 | blond doré cendré |
| 11 | 6-hydroxy 7-méthyl indole | 0,294 | p-phénylènediamine | 0,216 | blond doré cuivré |
| 12 | 6-hydroxy 7-méthyl indole | 0,588 | paraaminophénol | 0,438 | blond cuivré |
| 13 | 7-hydroxy 4-méthoxy 2,3-diméthylindole | 0,764 | paraaminophénol | 0,438 | blond clair beige nacré |
| 14 | 6-hydroxy 5-méthoxy 1-méthylindole | 0,708 | paraaminophénol | 0,438 | blond acajou nacré |
| 15 | 6-hydroxy 5-méthyl 2-carboxylindole | 0,76 | paraaminophénol | 0,438 | cuivré doré |

EXEMPLE DE TEINTURE N° 16

On prépare le mélange tinctorial suivant :

| | |
|---|---|
| - 5-acétamido 2,3-diméthyl 6-hydroxyindole | 0,545 g |
| - Paraphénylènediamine | 0,270 g |
| - Alcool oléique polyglycérolé à 2 moles de glycérol | 4,5 g |
| - Alcool oléique polyglycérolé à 4 moles de glycérol | 4,5 g |
| - ETHOMEEN O 12 - Société ARMOON HESS CHEMICAL Ltd (oléylamine oxyéthylénée à 12 moles d'oxyde d'éthylène) | 4,5 g |
| - COMPERLAN KD - Société HENKEL (diéthanolamide de coprah) | 9,0 g |
| - Propylèneglycol | 4,0 g |
| - 2-butoxyéthanol | 8,0 g |
| - Ethanol à 96° | 6,0 g |
| - MASQUOL DTPA - Société PROTEX (sel pentasodique de l'acide diéthylène triamine pentacétique) | 2,0 g |
| - Hydroquinone | 0,15 g |
| - Solution de bisulfite de sodium à 35° Bé | 1,3 g |
| - Ammoniaque à 22° Bé | 10,0 g |
| - Eau | qsp 100,0 g |

Au moment de l'emploi, on ajoute poids à poids une solution d'eau oxygénée à 20 volumes. Le mélange, appliqué 30 minutes à température ambiante sur cheveux naturels à 90% de blancs leur confère, après shampooing et rinçage, une coloration châtain.

EXEMPLE

Synthèse du 5-acétylamino 2,3-diméthyl 6-hydroxyindole.

A une solution de 50,7 g de 5-amino 2,3-diméthyl 6-hydroxyindole dans 250 ml d'eau, on ajoute une solution de 41,3 g de sulfite de sodium dans 130 ml d'eau à température ambiante.

On additionne en une seule fois 15,6 ml d'anhydride acétique. Après une heure d'agitation, on refroidit la suspension, filtre le précipité. On essore le solide jusqu'à pH neutre et on le sèche.

L'analyse du produit, recristallisé de l'isopropanol, donne les résultats suivants :

pF = 217°C

| Analyse élémentaire pour $C_{12}H_{14}N_2O_2$ | | | | |
|---|---|---|---|---|
| | C | H | N | O |
| Calculé | 66,03 | 6,47 | 12,84 | 14,66 |
| Trouvé | 65,90 | 6,50 | 12,67 | 14,93 |

**Revendications**

**Revendications pour les Etats contractants suivants : AT, BE, CH, DE, DK, FR, GB, GR, IT, LI, NL, SE**

**1.** Procédé de teinture des fibres kératiniques, en particulier des cheveux humains, caractérisé en ce que l'on applique sur celles-ci une composition comportant au moins un composé répondant à la formule :

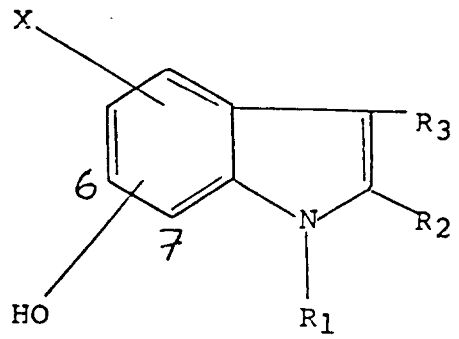

(I)

dans laquelle $R_1$ désigne un atome d'hydrogène, un radical alkyle en $C_1$-$C_4$; $R_2$ et $R_3$, identiques ou différents, désignent un atome d'hydrogène, un radical alkyle inférieur en $C_1$-$C_4$, un radical carboxyle ou un radical alcoxycarbonyle; X désigne un radical alkyle inférieur en $C_1$-$C_4$, un radical alcoxy en $C_1$-$C_{18}$, un atome d'halogène, un radical acyloxy en $C_1$-$C_{20}$, un groupement acétylamino, un groupement triméthylsilyloxy, ou un groupement dialkyl ($C_1$-$C_4$) aminométhyl; le groupement OH occupant les positions 6 ou 7, ainsi que leurs sels, comme coupleur, et au moins un précurseur de colorants d'oxydation para et/ou ortho. et en ce que l'on applique sur celles-ci un agent oxydant contenant du peroxyde d'hydrogène, l'agent oxydant étant soit introduit juste avant l'emploi dans la composition comportant ledit composé de formule (I) et le précurseur de colorant d'oxydation soit additionné sur les fibres, sans application d'ion iodure.

**2.** Procedé selon la revendication 1, caractérisé par le fait que les composés de formule (I) sont choisis parmi le 6-hydroxy 5-acétoxyindole, le 6-hydroxy 5-méthoxyindole, le 6-hydroxy 5-méthoxy 2-méthylindole, le 6-hydroxy 5-butoxyindole, l'acide 6-hydroxy 5-méthoxyindole 2-carboxylique, le 6-hydroxy 7-méthoxyindole, le 6-hydroxy 5-méthoxy 2,3-diméthyl indole, le 7-hydroxy 4-méthoxy 2,3-diméthylindole, le 6-hydroxy 5-tétradécanoyloxyindole, le 6-hydroxy 5-hexadécyloxyindole, le 6-hydroxy 5-butanoyloxyindole, le 6-hydroxy 5-oléyloxy indole, le 6-hydroxy 5-éthanoyloxy indole, le 6-hydroxy 5-hexanoyloxyindole, le 6-hydroxy 5-pivaloyloxyindole, le 6-hydroxy 5-formyloxyindole, le 6-hydroxy 5-triméthyl silyloxyindole, le 6-hydroxy 7-méthylindole, le 7-hydroxy 6-diméthylaminométhylindole, le 7-hydroxy 6-méthoxy 2-méthylindole, le 6-hydroxy 7-méthoxy 2-éthoxy carbonylindole, le 6-hydroxy 7-diméthylamino méthylindole, le 6-hydroxy 3,7-diméthylindole, le 6-hydroxy 3-méthyl 7-diméthylaminométhylindole, le 6-hydroxy 5-méthoxy 1-méthylindole, le 6-hydroxy 5-méthyl -2-carboxylindole, le 6-hydroxy 7-méthoxy 2-méthylindole, le 6-hydroxy 5-méthoxy 2-méthylindole, le 6-hydroxy 5-acétylamino 2,3-diméthylindole.

**3.** Procédé selon l'une quelconque des revendications 1 ou 2, caractérisé par le fait que les précurseurs de colorants d'oxydation para sont choisis parmi les paraphénylènediamines, les para-aminophénols, les précurseurs hétérocycliques para, les bases doubles.

**4.** Procédé selon l'une quelconque des revendications 1 à 3, caractérisé par le fait que les paraphénylènediamines sont choisies parmi les composés répondant à la formule (II) :

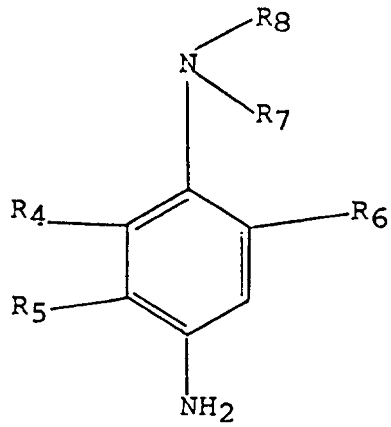

(II)

dans laquelle $R_4$, $R_5$ et $R_6$, identiques ou différents, représentent un atome d'hydrogène ou d'halogène, un radical alkyle ayant 1 à 4 atones de carbone, un radical alcoxy ayant 1 à 4 atomes de carbone, $R_7$ et $R_8$, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, pipéridinoalkyle, morpholinoalkyle, dans lesquels les groupements alkyle ou alcoxy ont de 1 à 4 atomes de carbone, ou bien $R_7$ et $R_8$ forment, conjointement avec l'atome d'azote auquel ils sont liés, un hétérocycle pipéridino ou morpholino, sous réserve que $R_4$ ou $R_6$ représente un atome d'hydrogène lorsque $R_7$ et $R_8$ ne représentent pas un atome d'hydrogène, ainsi que les sels de ces composés.

**5.** Procédé selon la revendication 4, caractérisé par le fait que les composés de formule (II) sont choisis parmi la p-phénylènediamine, la p-toluylènediamine, la méthoxyparaphénylènediamine, la chloroparaphénylènediamine, la 2,6-diméthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di(β-hydroxyéthyl)-paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino)-phényl] morpholine, la N-[(4'-amino)phényl]pipéridine et leurs sels.

**6.** Procédé selon l'une quelconque des revendications 1 à 4, caractérisé par le fait que les les p-aminophénols sont choisis parmi le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)-4-aminophénol, le 2-méthoxy 4-aminophénol, le 3-méthoxy 4-aminophénol, le 2-méthoxyméthyl 4-aminophénol, le 2,5-diméthyl 4-aminophénol.

**7.** Procédé selon l'une des revendications 1 et 2, caractérisé par le fait que les précurseurs de colorants d'oxydation de type ortho sont choisis parmi les orthoaminophénols et les orthophénylènediamines.

**8.** Procédé selon la revendication 3 caractérisé par le fait que les bases dites doubles sont des bis-phénylalkylènediamines, répondant à la formule :

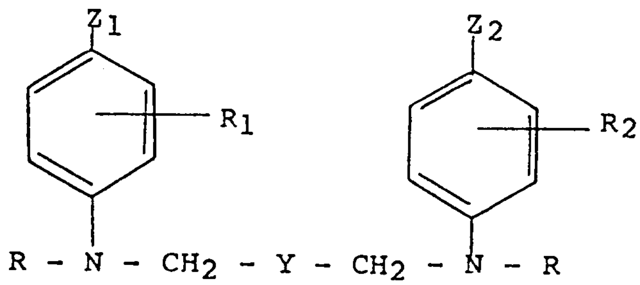

(III)

dans laquelle :

$Z_1$ et $Z_2$, identiques ou différents, représentent des groupements hydroxyle ou $NHR_3$, où $R_3$désigne un atome d'hydrogène ou un radical alcoyle inférieur;

$R_1$ et $R_2$, identiques ou différents, représentent soit des atomes d'hydrogène, soit des atomes d'halogène, soit encore des groupements alcoyle;

R représente un atome d'hydrogène, un groupe alcoyle, hydroxyalcoyle ou aminoalcoyle, dont le reste amino peut être substitué;

Y représente un radical pris dans le groupe constitué par les radicaux suivants : $-(CH_2)_n-$, $(CH_2)_n$, $-O(CH_2)_{n'}-$, $-(CH_2)_{n'}-CHOH-(CH_2)_{n'}-(CH_2)_n$,

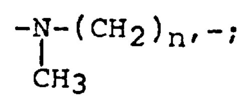

<u>n</u> étant un nombre entier compris entre O et 8 et <u>n</u>' un nombre entier compris entre O et 4, cette base pouvant se présenter sous forme de ses sels d'addition avec des acides.

**9.** Procédé selon la revendication 8, caractérisé par le fait que les composés de formule (III), sont choisis parmi le N,N'bis-(β-hydroxyéthyl) N,N'-bis(4'-aminophényl)1,3-diamino 2-propanol, la N,N'bis-(β-hydroxyéthyl)N,N'-bis(4'-aminophényl)éthylène diamine, la N,N'bis-(4-aminophényl)tétraméthylène diamine, la N,N'bis-(β-hydroxyéthyl) N,N'bis-(4-aminophényl)tétraméthylènediamine, la N,N'bis-(4-méthylaminophényl)tétraméthylène diamine, la N,N'bis-(éthyl)N,N'-bis(4'-amino 3'-méthylphényl)éthylènediamine.

**10.** Procédé selon l'une quelconque des revendications 1 à 9, caractérisé par le fait que l'on met également en oeuvre en plus des coupleurs hétérocycliques de formule (I), des métadiphénols, des métaaminophénols, des métaphénylènediamines, des métaacylaminophénols, des métauréidophénols, des métacarbalcoxyaminophénols, l'α-naphtol, des composés β-cétoniques, des pyrazolones ou le 4-hydroxyindole.

**11.** Procédé selon l'une quelconque des revendications 1 à 10, caractérisé par le fait que l'on utilise également des colorants directs.

**12.** Composition tinctoriale pour fibres kératiniques, caractérisée par le fait qu'elle contient dans un milieu approprié pour la teinture de ces fibres, au moins un composé répondant à la formule :

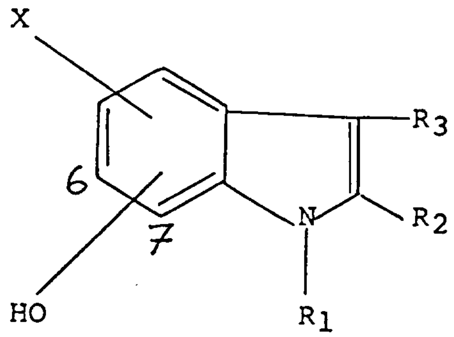

(I)

dans laquelle $R_1$ désigne un atome d'hydrogène, un radical alkyle en $C_1$-$C_4$; $R_2$ et $R_3$, identiques ou différents, désignent un atome d'hydrogène, un radical alkyle inférieur en $C_1$-$C_4$, un radical carboxyle ou un radical alcoxycarbonyle; X désigne un radical alkyle inférieur en $C_1$-$C_4$, un radical alcoxy en $C_1$-$C_{18}$, un atome d'halogène, un radical acyloxy en $C_1$-$C_{20}$, un groupement acétylamino, un groupement triméthylsilyloxy, ou un groupement dialkyl ($C_1$-$C_4$) aminométhyl; le groupement OH occupant les positions 6 ou 7, ainsi que leurs sels à titre de coupleur(s) et au moins un précurseur de colorant d'oxydation para et/ou ortho choisi parmi les paraaminophénols, les précurseurs hétérocycliques, les orthoaminophénols, les orthophénylènediamines, les bases dites doubles de la famille des bisphénylalkylènediamines, les paraphénylènediamines de formule :

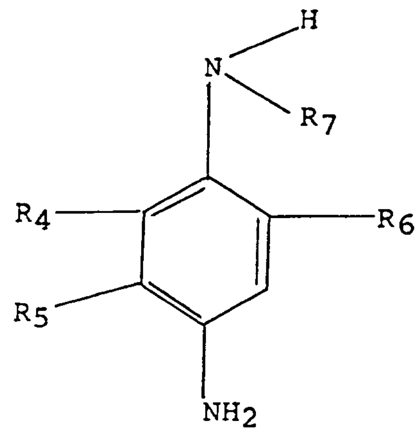

(IV)

dans laquelle $R_4$, $R_5$ et $R_6$, identiques ou différents, représentent un atome d'hydrogène ou d'halogène, un radical alkyle ayant 1 à 4 atomes de carbone, un radical alcoxy ayant 1 à 4 atomes de carbone, $R_7$ représente un atome d'hydrogène, un radical alkyle, carbamylalkyle, mésylaminoalkyle, acétylaminoalkyle, uréidoalkyle, carbalcoxyaminoalkyle, pipéridinoalkyle, morpholinoalkyle, ces groupes alkyle ou alcoxy ayant de 1 à 4 atomes de carbone ainsi que les sels de ces composés, cette composition ne comportant pas d'ion iodure.

**13.** Composition selon la revendication 12, caractérisée par le fait que les paraphénylènediamines sont choisies parmi la p-phénylènediamine, la p-toluylènediamine, la méthoxyparaphénylènediamine, la chloroparaphénylènediamine, la 2,6-diméthylparaphénylènediamine, la 2,5-diméthylparaphénylènediamine, la 2-méthyl 5-méthoxyparaphénylènediamine, la 2,6-diméthyl 5-méthoxyparaphénylènediamine.

**14.** Composition selon l'une quelconque des revendications 12 et 13, caractérisée par le fait que les colorants par oxydation de type para et/ou ortho et les coupleurs sont présents dans des proportions comprises entre 0,3 et 7% en poids par rapport au poids total de la composition.

**15.** Composition selon l'une quelconque des revendications 12 à 14, caractérisée par le fait que le composé de formule (I) est présent dans des proportions comprises entre 0,05 et 3,5% en poids par rapport au poids total de la composition.

**16.** Composition selon l'une quelconque des revendications 12 à 15, caractérisée par le fait que le milieu approprié pour la teinture de ces fibres est aqueux et qu'il a un pH compris entre 8 et 11.

**17.** Composition selon l'une quelconque des revendications 12 à 16, caractérisée par le fait que la composition contient également des agents tensio-actifs anioniques, cationiques, non ioniques, amphotères ou leurs mélanges.

**18.** Composition selon l'une quelconque des revendications 12 à 17, caractérisée par le fait que la composition contient également des solvants organiques dans des proportions comprises entre 1 et 40% en poids par rapport au poids total de la composition.

**19.** Composition selon l'une quelconque des revendications 12 à 18, caractérisée par le fait que la composition contient également des agents épaississants, des agents antioxydants, des agents de pénétration, des agents séquestrants, des tampons et/ou des parfums.

**20.** Composition selon l'une quelconque des revendications 12 à 19, caractérisée par le fait qu'elle se présente sous forme de liquides, de crèmes, de gels et qu'elle est éventuellement conditionnée en aérosols en présence d'un agent propulseur.

**21.** Procédé de teinture des fibres kératiniques, en particulier des cheveux humains selon l'une des revendications 1 á 11 caractérisé par le fait qu'on applique sur celles-ci au moins une composition telle que définie dans l'une des revendications 12 á 20, et au moins un agent oxydant contenant du peroxyde d'hydrogène, l'application durant 10 à 40 minutes et étant suivie d'un rinçage, éventuellement d'un lavage et d'un séchage.

**22.** Composé nouveau constitué par le 6-hydroxy 5-acétylamino 2,3-diméthylindole.

**Revendications pour l'Etat contractant suivant : ES**

**1.** Procédé de teinture de fibres kératiniques, en particulier des cheveux humains, caractérisé en ce que l'on applique sur celles-ci une composition comportant au moins d'un composé répondant à la formule :

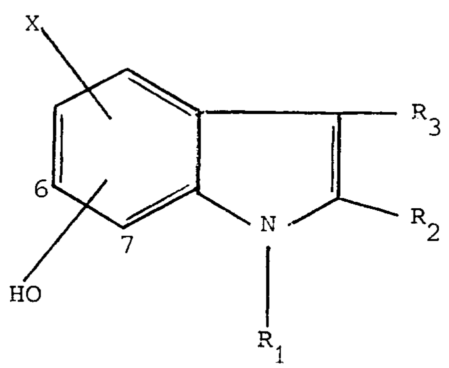

(I)

dans laquelle $R_1$ désigne un atome d'hydrogène, un radical alkyle en $C_1$-$C_4$; $R_2$ et $R_3$, identiques ou différents, désignent un atome d'hydrogène, un radical alkyle inférieur en $C_1$-$C_4$, un radical carboxyle ou un radical alcoxycarbonyle; X désigne un radical alkyle inférieur en $C_1$-$C_4$, un radical alcoxy en $C_1$-$C_{18}$, un atome d'halogène, un radical acyloxy en $C_1$-$C_{20}$, un groupement acétylamino, un groupement triméthylsilyloxy, ou un groupement dialkyl($C_1$-$C_4$)aminométhyl; le groupement OH occupant les positions 6 ou 7, ainsi que leurs sels comme coupleurs précurseur de colorants d'oxydation para et/ou ortho, et en ce que l'on applique sur celles-ci un agent oxydant contenant du peroxyde d'hydrogène, l'agent oxydant étant soit introduit juste avant l'emploi dans la composition comportant ledit composé de formule (I) et le précurseur de colorant d'oxydation, soit additionné sur les fibres, sans application d'ion iodure.

**2.** Procédé selon la revendication 1, caractérisée par le fait que les composes de formule (I) sont choisis parmi le 6-hydroxy 5-acétoxy indole, le 6-hydroxy 5-méthoxyindole, le 6-hydroxy 5-méthoxy 2-méthylindole, le 6-hydroxy 5-butoxyindole, l'acide 6-hydroxy 5-méthoxyindole 2-carboxylique, le 6-hydroxy 7-méthoxyindole, le 6-hydroxy 5-méthoxy 2,3-diméthylindole, le 7-hydroxy 4-méthoxy 2,3-diméthylindole, le 6-hydroxy 5-tétradécanoyloxyindole, le 6-hydroxy 5-hexadécyloxyindole, le 6-hydroxy 5-butanoyloxyindole, le 6-hydroxy 5-oléyloxyindole, le 6-hydroxy 5-éthanoyloxyindole, le 6-hydroxy 5-hexanoyloxyindole, le 6-hydroxy 5-pivaloyloxyindole, le 6-hydroxy 5-formyloxyindole, le 6-hydroxy 5-triméthylsilyloxyindole, le 6-hydroxy 7-méthylindole, le 7-hydroxy 6-diméthylaminométhylindole, le 7-hydroxy 6-méthoxy 2-méthylindole, le 6-hydroxy 7-méthoxy 2-éthoxycarbonylindole, le 6-hydroxy 7-diméthylamino méthylindole, le 6-hydroxy 3,7-diméthylindole, le 6-hydroxy 3-méthyl 7-diméthylaminométhylindole, le 6-hydroxy 5-méthoxy 1-méthylindole, le 6-hydroxy 5-méthyl 2-carboxylindole, le 6-hydroxy 7-méthoxy 2-méthylindole, le 6-hydroxy 5-méthoxy 2-méthylindole, le 6 hydroxy 5-acétylamino 2,3-diméthylindole.

**3.** Procédé selon l'une quelconque des revendications 1 ou 2, caractérisée par le fait que les précurseurs de colorants d'oxydation para sont choisis parmi les paraphénylènediamines, les para-amino-phénols, les précurseurs hétérocycliques para, les bases doubles.

**4.** Procédé selon l'une quelconque des revendications 1 à 3, caractérisée par le fait que les paraphénylènediamines sont choisies parmi les composés répondant à la formule (II) :

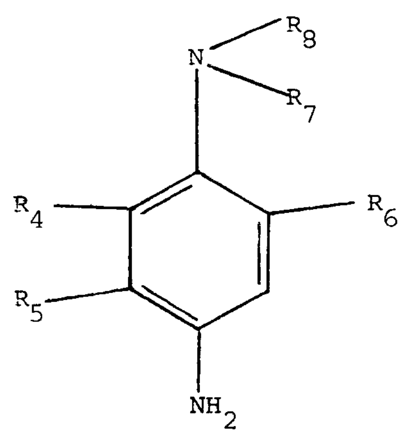

(II)

dans laquelle $R_4$, $R_5$ et $R_6$, identiques ou différents, représentent un atome d'hydrogène ou d'halogène, un radical alkyle ayant 1 à 4 atomes de carbone, un radical alcoxy ayant 1 à 4 atomes de carbone, $R_7$ et $R_8$, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, pipéridinoalkyle, morpholinoalkyle, dans lesquels les groupements alkyle ou alcoxy ont de 1 à 4 atomes de carbone, ou bien $R_7$ et $R_8$ forment, conjointement avec l'atome d'azote auquel ils sont liés, un hétérocycle pipéridino ou morpholino, sous réserve que $R_4$ ou $R_6$ représente un atome d'hydrogène lorsque $R_7$ et $R_8$ ne représentent pas un atome d'hydrogène, ainsi que les sels de ces composés.

**5.** Procédé selon la revendication 4, caractérisée par le fait que les composés de formule (II) sont choisis parmi la p-phénylènediamine, la p-toluylènediamine, la méthoxyparaphénylènediamine, la chloroparaphénylènediamine, la 2,6-diméthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di-($\beta$-hydroxyéthyl)-paraphénylènediamine, la 3-méthyl 4-amino N,N-di-($\beta$-hydroxyéthyl)aniline, la 3-chloro 4-amino N,N-di-($\beta$-hydroxyéthyl)aniline, la 4-amino N,N-(éthyl,carbamylméthyl) aniline, la 3-méthyl 4-amino N,N-(éthyl,carbamylméthyl)aniline, la 4-amino N,N-(éthyl,$\beta$-pipéridinoéthyl)aniline, la 3-méthyl 4-amino N,N-(éthyl,$\beta$-pipéridinoéthyl)aniline, la 4-amino N,N-(éthyl,$\beta$-morpholinoéthyl)aniline, la 3-méthyl 4-amino N,N-(éthyl,$\beta$-morpholinoéthyl) aniline, la 4-amino N,N-(éthyl,$\beta$-acétylaminoéthyl)aniline, la 4-amino N-($\beta$-méthoxyéthyl)aniline, la 3-méthyl 4-amino N,N-(éthyl,$\beta$-acétylaminoéthyl)aniline, la 4-amino N,N-(éthyl,$\beta$-mésylaminoéthyl)aniline, la 3-méthyl 4-amino N,N-(éthyl,$\beta$-mésylaminoéthyl)aniline, la 4-amino N,N-(éthyl,$\beta$-sulfoéthyl)aniline, la 3-méthyl 4-amino N,N-(éthyl,$\beta$-sulfoéthyl)aniline, la N-[(4'-amino)-

phényl]morpholine, la N-[(4'-amino)phényl]pipéridine et leurs sels.

**6.** Procédé selon l'une quelconque des revendications 1 à 4, caractérisée par le fait que les p-aminophénols sont choisis parmi le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-($\beta$-hydroxyéthyl)-4-aminophénol, le 2-méthoxy 4-aminophénol, le 3-méthoxy 4-aminophénol, le 2-méthoxyméthyl 4-aminophénol, le 2,5-diméthyl 4-aminophénol.

**7.** Procédé selon l'une des revendications 1 et 2, caractérisée par le fait que les précurseurs de colorants d'oxydation de type ortho sont choisis parmi les orthoaminophénols et les orthophénylènediamines.

**8.** Procédé selon la revendication 3, caractérisée par le fait que les bases dites doubles sont des bis-phénylalkylènediamines, répondant à la formule :

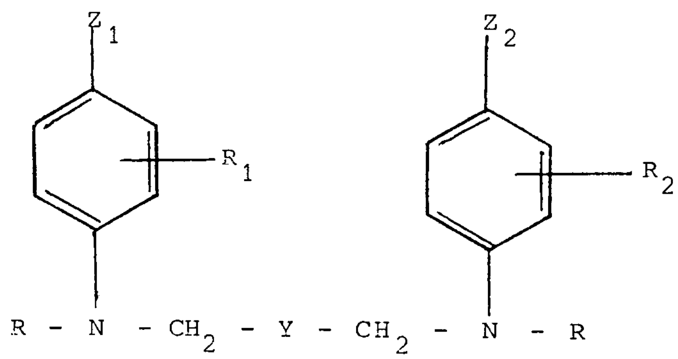

(III)

dans laquelle :

$Z_1$ et $Z_2$, identiques ou différents, représentent des groupements hydroxyle ou $NHR_3$, où $R_3$ désigne un atome d'hydrogène ou un radical alcoyle inférieur;

$R_1$ et $R_2$, identiques ou différents, représentent soit des atomes d'hydrogène, soit des atomes d'halogène, soit encore des groupements alcoyle;

R représente un atome d'hydrogène, un groupe alcoyle, hydroxyalcoyle ou aminoalcoyle, dont le reste amino peut être substitué;

Y représente un radical pris dans le groupe constitué par les radicaux suivants : $-(CH_2)_n-$, $(CH_2)_{n'}$ -O-$(CH_2)_{n'}$-, $-(CH_2)_{n'}$ -CHOH-$(CH_2)_{n'}$ $-(CH_2)_{n'}$

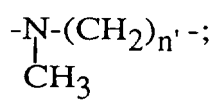

n étant un nombre entier compris entre O et 8 et n' un nombre entier compris entre O et 4, cette base pouvant se présenter sous forme de ses sels d'addition avec des acides.

**9.** Procédé selon la revendication 8, caractérisée par le fait que les composés de formule (III) sont choisis parmi le N,N'-bis-($\beta$-hydroxyéthyl)N,N'-bis-(4'-aminophényl) 1,3-diamino 2-propanol, la N,N'-bis-($\beta$-hydroxyéthyl)N,N'-bis-(4'-aminophényl)éthylènediamine, la N,N'-bis-(4-aminophényl)-tétraméthylènediamine, la N,N'-bis-($\beta$-hydroxyéthyl)N,N'-bis-(4-aminophényl)tétraméthylènediamine, la N,N'-bis-(4-méthylaminophényl)tétraméthylènediamine, la N,N'-bis-(éthyl)N,N'-bis-(4'-amino 3'-méthylphényl)éthylènediamine.

**10.** Procédé selon l'une quelconque des revendications 1 à 9, caractérisée par le fait que l'on met également en oeuvre en plus des coupleurs hétérocycliques de formule (I), des métadiphénols, des métaaminophénols, des métaphénylènediamines, des métaacylaminophénols, des métauréidophénols, des métacarbalcoxyaminophénols, l'$\alpha$-naphtol, des composés $\beta$-cétoniques, des pyrazolones ou le 4-hydroxyindole.

**11.** Procédé selon l'une quelconque des revendications 1 à 10, caractérisée par le fait que l'on utilise également des colorants directs.

**12.** Composition tinctoriale pour fibres kératiniques, caractérisée par le fait qu'elle contient dans un milieu approprié pour la teinture de ces fibres, au moins un composé répondant à la formule :

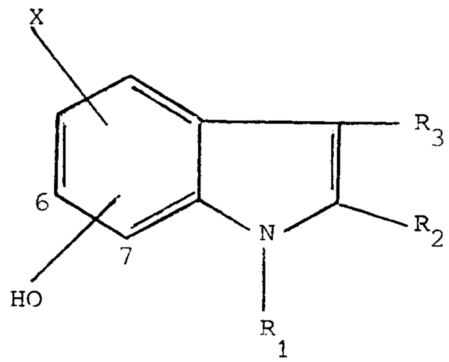

(I)

dans laquelle $R_1$ désigne un atome d'hydrogène, un radical alkyle en $C_1$-$C_4$; $R_2$ et $R_3$, identiques ou différents, désignent un atome d'hydrogène, un radical alkyle inférieur en $C_1$-$C_4$, un radical carboxyle ou un radical alcoxycarbonyle; X désigne un radical alkyle inférieur en $C_1$-$C_4$, un radical alcoxy en $C_1$-$C_{18}$, un atome d'halogène, un radical acyloxy en $C_1$-$C_{20}$, un groupement acétylamino, un groupement triméthylsilyloxy, ou un groupement dialkyl($C_1$-$C_4$)aminométhyl; le groupement OH occupant les positions 6 ou 7, ainsi que leurs sels à titre de coupleur(s) et au moins un précurseur de colorant d'oxydation para et/ou ortho choisi parmi les paraaminophénols, les précurseurs hétérocycliques, les orthoaminophénols, les orthophénylènediamines, les bases dites doubles de la famille des bisphénylalkylènediamines, les paraphénylènediamines de formule :

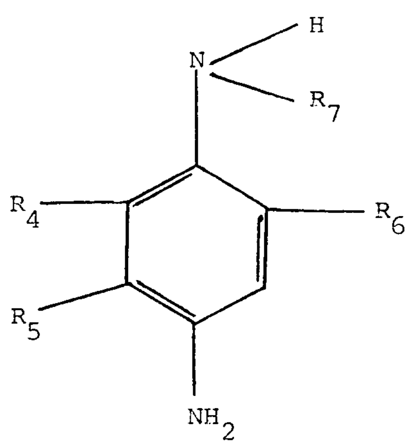

(IV)

dans laquelle $R_4$, $R_5$ et $R_6$, identiques ou différents, représentent un atome d'hydrogène ou d'halogène, un radical alkyle ayant 1 à 4 atomes de carbone, un radical alcoxy ayant 1 à 4 atomes de carbone, $R_7$ représente un atome d'hydrogène, un radical alkyle, carbamylalkyle, mésylaminoalkyle, acétylaminoalkyle, uréidoalkyle, carbalcoxyaminoalkyle, pipéridinoalkyle, morpholinoalkyle, ces groupes alkyle ou alcoxy ayant de 1 à 4 atomes de carbone, ainsi que les sels de ces composés, cette composition ne comportant pas d'ion iodure.

**13.** Composition selon la revendication 12, caractérisée par le fait que les paraphénylènediamines sont choisies parmi la p-phénylène diamine, la p-toluylènediamine, la méthoxyparaphénylènediamine, la chloroparaphénylènediamine, la 2,6-diméthylparaphénylènediamine, la 2,5-diméthylparaphénylènediamine, la 2-méthyl 5-méthoxyparaphénylènediamine, la 2,6-diméthyl 5-méthoxyparaphénylènediamine.

**14.** Composition selon l'une quelconque des revendications 12 et 13, caractérisée par le fait que les colorants par oxydation de type para et/ou ortho et les coupleurs sont présents dans des proportions comprises entre 0,3 et 7% en poids par rapport au poids total de la composition.

**15.** Composition selon l'une quelconque des revendications 12 à 14, caractérisée par le fait que le composé de formule (I) est présent dans des proportions comprises entre 0,05 et 3,5% en poids par rapport au poids total de la composition.

**16.** Composition selon l'une quelconque des revendications 12 à 15, caractérisée par le fait que le milieu approprié pour la teinture de ces fibres est aqueux et qu'il a un pH compris entre 8 et 11.

**17.** Composition selon l'une quelconque des revendications 12 à 16, caractérisée par le fait que la composition contient également des agents tensio-actifs anioniques, cationiques, non-ioniques, amphotères ou leurs mélanges.

**18.** Composition selon l'une quelconque des revendications 12 à 17, caractérisée par le fait que la composition contient également des solvants organiques dans des proportions comprises entre 1 et 40% en poids par rapport au poids total de la composition.

**19.** Composition selon l'une quelconque des revendications 12 à 18, caractérisée par le fait que la composition contient également des agents épaississants, des agents antioxydants, des agents de pénétration, des agents séquestrants, des tampons et/ou des parfums.

**20.** Composition selon l'une quelconque des revendications 12 à 19, caractérisée par le fait qu'elle se présente sous forme de liquides, de crèmes, de gels et qu'elle est éventuellement conditionnée en aérosols en présence d'un agent propulseur.

**21.** Procédé de teinture des fibres kératiniques, en particulier des cheveux humains selon l'une des revendications 1 à 11, caractérisé par le fait qu'on applique sur celles-ci au moins une composition telle que définie dans l'une des revendications 12 à 20, et au moins un agent oxydant contenant du peroxyde d'hydrogène, l'application durant 10 à 40 minutes et étant suivie d'un rinçage, éventuellement d'un lavage et d'un séchage.

**22.** Procédé de préparation du 6-hydroxy 5-acétylamino 2,3-diméthylindole, caractérisé en ce que l'on mélange du 5-amino 2,3-diméthyl 6-hydroxyindole à du sulfite de sodium, on additionne de l'anhydride acétique, et on récupère le composé du titre.

**23.** Procédé de préparation d'une composition destinée à être utilisée pour la teinture des fibres kératiniques, caractérisé par le fait que l'on introduit, dans un milieu approprié pour la teinture, au moins un composé répondant à la formule :

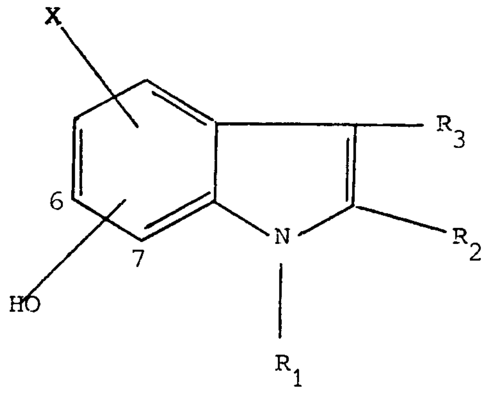

(I)

dans laquelle $R_1$ désigne un atome d'hydrogène, un radical alkyle en $C_1$-$C_4$; $R_2$ et $R_3$, identiques ou différents, désignent un atome d'hydrogène, un radical alkyle inférieur en $C_1$-$C_4$, un radical carboxyle

ou un radical alcoxycarbonyle; X désigne un radical alkyle inférieur en $C_1$-$C_4$, un radical alcoxy en $C_1$-$C_{18}$, un atome d'halogène, un radical acyloxy en $C_1$-$C_{20}$, un groupement acétylamino, un groupement triméthylsilyloxy, ou un groupement dialkyl($C_1$-$C_4$)aminométhyl; le groupement OH occupant les positions 6 ou 7, ainsi que leurs sels à titre de coupleur(s) et au moins un précurseur de colorant d'oxydation para et/ou ortho choisi parmi les paraaminophénols, les précurseurs hétérocycliques, les orthoaminophénols, les orthophénylènediamines, les bases dites doubles de la famille des bisphénylalkylènediamines, les paraphénylènediamines de formule :

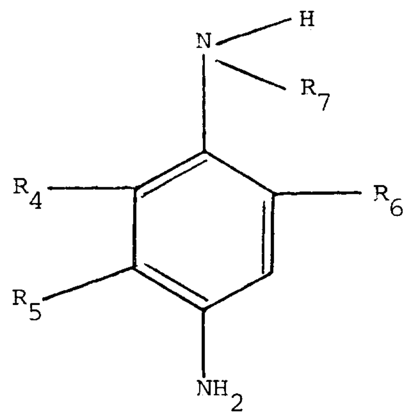

(IV)

dans laquelle $R_4$, $R_5$ et $R_6$, identiques ou différents, représentent un atome d'hydrogène ou d'halogène, un radical alkyle ayant 1 à 4 atomes de carbone, un radical alcoxy ayant 1 à 4 atomes de carbone, $R_7$ représente un atome d'hydrogène, un radical alkyle, carbamylalkyle, mésylaminoalkyle, acétylaminoalkyle, uréidoalkyle, carbalcoxyaminoalkyle, pipéridinoalkyle, morpholinoalkyle, ces groupes alkyle ou alcoxy ayant de 1 à 4 atomes de carbone, ainsi que les sels de ces composés, dans des proportions comprises entre 0,3 et 7% en poids par rapport au poids total de la composition.

**Claims**

**Claims for the following Contracting States : AT, BE, CH, DE, DK, FR, GB, GR, IT, LI, NL, SE**

1. Method for dyeing keratinous fibres, in particular human hair, characterized by applying thereto a composition comprising at least one compound corresponding to the formula:

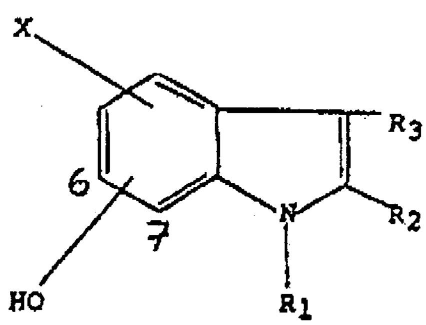

(I)

in which $R_1$ denotes a hydrogen atom or a $C_1$-$C_4$ alkyl radical; $R_2$ and $R_3$, which may be identical or different, denote a hydrogen atom, a $C_1$-$C_4$ lower alkyl radical, a carboxyl radical or an alkoxycarbonyl radical; X denotes a $C_1$-$C_4$ lower alkyl radical, a $C_1$-$C_{18}$ alkoxy radical, a halogen atom, a $C_1$-$C_{20}$ acyloxy radical, an acetylamino group, a trimethylsilyloxy group or a dialkyl ($C_1$-$C_4$) aminomethyl group; the OH group occupying the 6- or 7-positions, as well as their salts, as coupler, and at least one para and/or ortho oxidation dye precursor, and by applying thereto an oxidizing agent containing hydrogen peroxide, the oxidizing agent either being introduced just before use into the composition comprising said compound of formula (I) and the oxidation dye precursor, or being added to the fibres, without application of iodide ion.

2. Method according to Claim 1, characterized in that the compounds of formula (I) are chosen from 6-hydroxy-5-acetoxyindole, 6-hydroxy-5-methoxyindole, 6-hydroxy-5-methoxy-2-methylindole, 6 hydroxy-5-butoxyindole, 6-hydroxy-5-methoxyindole-2-carboxylic acid, 6-hydroxy-7-methoxyindole, 6-hydroxy-5-methoxy-2,3-dimethylindole, 7-hydroxy-4-methoxy-2,3-dimethylindole, 6-hydroxy-5-tetradecanoyloxyindole, 6-hydroxy-5-hexadecyloxyindole, 6-hydroxy-5-butanoyloxyindole, 6-hydroxy-5-oleyloxyindole, 6-hydroxy-5-ethanoyloxyindole, 6-hydroxy-5-hexanoyloxyindole, 6-hydroxy-5-pivaloyloxyindole, 6-hydroxy-5-formyloxyindole, 6-hydroxy-5-trimethylsilyloxyindole, 6-hydroxy-7-methylindole, 7-hydroxy-6-dimethylaminomethylindole, 7-hydroxy-6-methoxy-2-methylindole, 6-hydroxy-7-methoxy-2-ethoxycarbonylindole, 6-hydroxy-7-dimethylaminomethylindole, 6-hydroxy-3,7-dimethylindole, 6-hydroxy-3-methyl-7-dimethylaminomethylindole, 6-hydroxy-5-methoxy-1-methylindole, 6-hydroxy-5-methyl-2-carboxyindole, 6-hydroxy-7-methoxy-2-methylindole, 6-hydroxy-5-methoxy-2-methylindole and 6-hydroxy-5-acetylamino-2,3-dimethylindole.

3. Method according to either of Claims 1 or 2, characterized in that the para oxidation dye precursors are chosen from para-phenylenediamines, para-aminophenols, para heterocyclic precursors and double bases.

4. Method according to any one of Claims 1 to 3, characterized in that the para-phenylenediamines are chosen from the compounds corresponding to the formula (II):

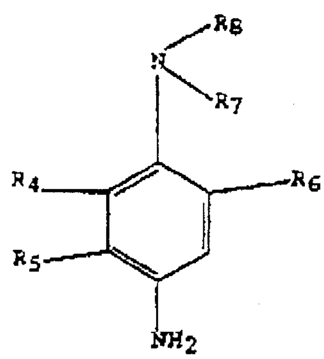

(II)

in which $R_4$, $R_5$ and $R_6$, which may be identical or different, represent a hydrogen or halogen atom, an alkyl radical having 1 to 4 carbon atoms or an alkoxy radical having 1 to 4 carbon atoms, and $R_7$ and $R_8$, which may be identical or different, represent a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl or morpholinoalkyl, in which the alkyl or alkoxy groups have from 1 to 4 carbon atoms, or $R_7$ and $R_8$ form, together with the nitrogen atom to which they are bonded, a piperidino or morpholino heterocycle, with the proviso that $R_4$ or $R_6$ represents a hydrogen atom when $R_7$ and $R_8$ do not represent a hydrogen atom, as well as the salts of these compounds.

5. Method according to Claim 4, characterized in that the compounds of formula (II) are chosen from p-phenylenediamine, p-tolylenediamine, methoxy-para-phenylenediamine, chloro-para-phenylenediamine, 2,6-dimethyl-para-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethyl-aniline, N,N-di(β-hydroxyethyl)-para-phenylenediamine, 3-methyl-4-amino-N,N-di(β-hydroxyethyl)aniline, 3-chloro-4-amino-N,N-di (β-hydroxyethyl)aniline, 4-amino-N,N-(ethyl,carbamylmethyl)aniline, 3-methyl-4-amino-N,N-(ethyl,carbamylmethyl)aniline, 4-amino-N,N-(ethyl, β-piperidinoethyl)aniline, 3-methyl-4-amino-N,N-(ethyl, β-piperidinoethyl) aniline, 4-amino-N,N- (ethyl, β-morpholinoethyl)aniline, 3-methyl-4-amino-N,N-(ethyl, β-morpholinoethyl)aniline, 4-amino-N,N- (ethyl, β-acetylaminoethyl)aniline, 4-amino-N-(β-methoxyethyl)aniline, 3-methyl-4-amino-N,N-(ethyl, β-acetylaminoethyl)aniline, 4-amino-N,N-(ethyl, β-mesylaminoethyl)aniline, 3-methyl-4-amino-N,N-(ethyl, β-mesylaminoethyl)aniline, 4-amino-N,N-(ethyl, β-sulphoethyl)aniline, 3-methyl-4-amino-N,N-(ethyl, β-sulphoethyl)aniline, N-[(4'-amino)phenyl]-morpholine and N-[(4'-amino)phenyl]piperidine, and their salts.

6. Method according to any one of Claims 1 to 4, characterized in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3, 5-dimethyl-4-aminophenol, 2,3-dimethyl-4-aminophenol, 2-hydroxymethyl-4-aminophenol, 2-($\beta$-hydroxyethyl)-4-aminophenol, 2-methoxy-4-aminophenol, 3-methoxy-4-aminophenol, 2-methoxymethyl-4-aminophenol and 2,5-dimethyl-4-aminophenol.

7. Method according to one of Claims 1 and 2, characterized in that the oxidation dye precursors of ortho type are chosen from ortho-aminophenols and ortho-phenylenediamines.

8. Method according to Claim 3, characterized in that the so-called double bases are bisphenylalklenediamines corresponding to the formula:

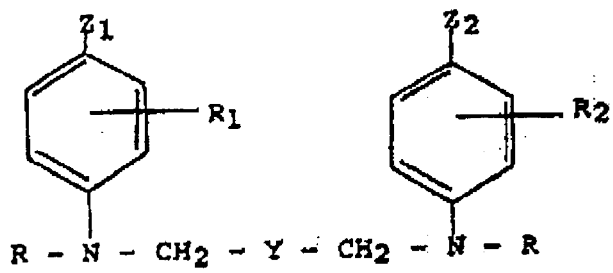

(III)

in which:

$Z_1$ and $Z_2$, which may be identical or different, represent hydroxyl or $NHR_3$ groups, where $R_3$ denotes a hydrogen atom or a lower alkyl radical;

$R_1$ and $R_2$, which may be identical or different, represent either hydrogen atoms or halogen atoms or alkyl groups;

R represents a hydrogen atom or an alkyl, hydroxyalkyl or aminoalkyl group, in which the amino radical may be substituted; and

Y represents a radical taken from the group comprising the following radicals: $-(CH_2)_n-$, $(CH_2)_{n'}$ -O-$(CH_2)_{n'}-$, $-(CH_2)_{n'}$-CHOH-$(CH_2)_{n'}$-$(CH_2)_{n'}$

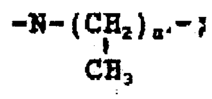

n being an integer between 0 and 8 and n' being an integer between 0 and 4, it being possible for this base to be in the form of its addition salts with acids.

9. Method according to Claim 8, characterized in that the compounds of formula (III) are chosen from N,N'-bis($\beta$-hydroxyethyl)-N,N'-bis (4'-aminophenyl)-1,3-diamino-2-propanol, N,N'-bis($\beta$-hydroxyethyl)-N,N'-bis(4'-aminophenyl)ethylenediamine, N,N'-bis(4-aminophenyl)tetramethylenediamine, N,N' -bis($\beta$-hydroxyethyl)-N,N'-bis(4-aminophenyl)tetramethylenediamine, N,N'-bis(4-methylaminophenyl)-tetramethylenediamine and N,N'-bis(ethyl)-N,N'-bis(4'-amino-3'-methylphenyl)ethylenediamine.

10. Method according to any one of Claims 1 to 9, characterized in that, in addition to the heterocyclic couplers of formula (I), meta-diphenols, meta-aminophenols, meta-phenylenediamines, meta-acylaminophenols, meta-ureidophenols, meta-carbalkoxyaminophenols, $\alpha$-naphthol, $\beta$-ketone compounds, pyrazolones or 4-hydroxyindole are also used.

11. Method according to any one of Claims 1 to 10, characterized in that direct dyes are-also used.

12. Tinctorial composition for keratinous fibres, characterized in that it contains, in a medium appropriate for dyeing those fibres, at least one compound corresponding to the formula:

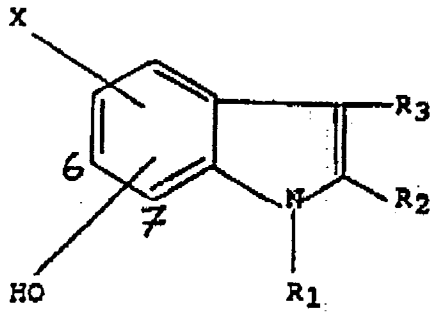

(I)

in which $R_1$ denotes a hydrogen atom or a $C_1$-$C_4$ alkyl radical; $R_2$ and $R_3$, which may be identical or different, denote a hydrogen atom, a $C_1$-$C_4$ lower alkyl radical, a carboxyl radical or an alkoxycarbonyl radicals; X denotes a $C_1$-$C_4$ lower alkyl radical, a $C_2$-$C_{18}$ alkoxy radical, a halogen atom, a $C_1$-$C_{20}$ acyloxy radical, an acetylamino group, a trimethylsilyloxy group or a dialkyl($C_1$-$C_4$)-aminomethyl group; the group OH occupying the 6- or 7-positions, as well as their salts as coupler(s) and at least one para and/or ortho oxidation dye precursor chosen from para-aminophenols, heterocyclic precursors, ortho-aminophenols, ortho-phenylenediamines, the so-called double bases of the family of bisphenylalklenediamines, and para-phenylenediamines of formula:

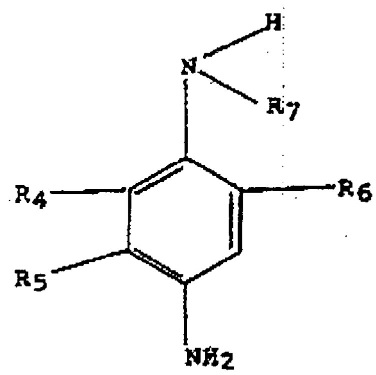

(IV)

in which $R_4$, $R_5$ and $R_6$, which may be identical or different, represent a hydrogen or halogen atom, an alkyl radical having 1 to 4 carbon atom or an alkoxy radical having 1 to 4 carbon atoms, and $R_7$ represents a hydrogen atom or an alkyl, carbamylalkyl, mesylaminoalkyl, acetylaminoalkyl, ureidoalkyl, carbalkoxyaminoalkyl, piperidinoalkyl or morpholinoalkyl radical, these alkyl or alkoxy groups having from 1 to 4 carbon atoms, as well as the salts of these compounds, this composition containing no iodide ion.

**13.** Composition according to Claim 12, characterized in that the para-phenylenediamines are chosen from p-phenylenediamine, p-tolylenediamine, methoxy-para-phenylenediamine, chloro-para-phenylenediamine, 2,6-dimethyl-para-phanylenediamine, 2,5-dimethyl-para-phenylenediamine, 2-methyl-5-methoxy-para-phenylenediamine and 2,6-dimethyl-5-methoxy-para-phenylenediamine.

**14.** Composition according to either of Claims 12 and 13, characterized in that the oxidation dyes of para and/or ortho type and the couplers are present in proportions of between 0.3 and 7% by weight relative to the total weight of the composition.

**15.** Composition according to any one of Claims 12 to 14, characterized in that the compound of formula (I) is present in proportions of between 0.05 and 3.5% by weight relative to the total weight of the composition.

**16.** Composition according to any one of Claims 12 to 15, characterized in that the medium appropriate for dyeing these fibres is aqueous and in that it has a pH of between 8 and 11.

**17.** Composition according to any one of Claims 12 to 16, characterized in that the composition also contains anionic, cationic, nonionic or amphoteric surface-active agents or their mixtures.

**18.** Composition according to any one of Claims 12 to 17, characterized in that the composition also contains organic solvents in proportions of between 1 and 40% by weight relative to the total weight of the composition.

**19.** Composition according to any one of Claims 12 to 18, characterized in that the composition also contains thickeners, antioxidants, penetrating agents, sequestering agents, buffers and/or perfumes.

**20.** Composition according to any one of Claims 12 to 19, characterized in that it is in the form of liquids, creams or gels and in, that it is optionally packaged as aerosols in the presence of a propellant.

**21.** Method for dyeing keratinous fibres, in particular human hair, according to one of Claims 1 to 11, characterized in that at least one composition as defined in one of Claims 12 to 20 and at least one oxidizing agent containing hydrogen peroxide are applied thereto, the application lasting 10 to 40 minutes and being followed by rinsing, if appropriate washing and drying.

**22.** Now compound consisting of 6-hydroxy-5-acetylamino-2,3-dimethylindole.

**Claims for the following Contracting State : ES**

**1.** Method for dyeing karatinous fibres, in particular human hair, characterized by applying thereto a composition comprising at least one compound corresponding to the formula:

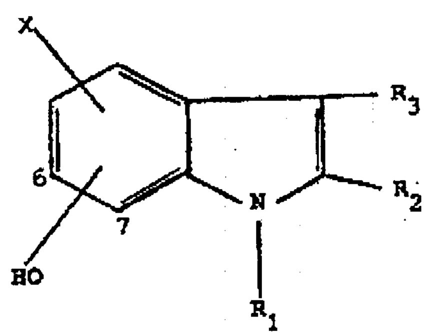

(I)

in which $R_1$ denotes a hydrogen atom or a $C_1$-$C_4$ alkyl radical; $R_2$ and $R_3$, which my be identical or different, denote a hydrogen atom, a $C_1$-$C_4$ lower alkyl radical, a carboxyl radical or an alkoxycarbonyl radical; X denotes a $C_1$-$C_4$ lower alkyl radical, a $C_1$-$C_{18}$ alkoxy radical, a halogen atom, a $C_1$-$C_{20}$ acyloxy radical, an acetylamino group, a trimethylsilyloxy group or a dialkyl($C_1$-$C_4$)aminomethyl group; the OH group occupying the 6- or -7 positions, as well as their salts as couplers [lacuna] para and/or ortho oxidation dye precursor, and by applying thereto an oxidizing agent containing hydrogen peroxide, the oxidizing agent either being introduced just before use into the composition comprising said compound of formula (I) and the oxidation dye precursor, or being added to the fibres, without application of iodide ion.

**2.** Method according to Claim 1, characterized in that the compounds of formula (I) are chosen from 6-hydroxy-5-acetoxyindole, 6-hydroxy-5-methoxyindole, 6-hydroxy-5-methoxy-2-methylindole, 6-hydroxy-5-butoxyindole, 6-hydroxy-5-methoxyindole-2-carboxylic acid, 6-hydroxy-7-methoxyindole, 6-hydroxy-5-methoxy-2,3-dimethylindole, 7-hydroxy-4-methoxy-2,3-dimethylindole, 6-hydroxy-5-tatradecanoyloxyindole, 6-hydroxy-5-hexadecyloxyindole, 6-hydroxy-5-butanoyloxyindole, 6-hydroxy-5-oleyloxyindole, 6-hydroxy-5-ethanoyloxyindole, 6-hydroxy-5-hexanoyloxyindole, 6-hydroxy-5-pivaloyloxyindole, 6-hydroxy-5-formyloxyindole, 6-hydroxy-5-tri-methylsilyloxyindole, 6-hydroxy-7-methylindole, 7-hydroxy-6-dimethylaminomethylindole, 7-hydroxy-6-methoxy-2-methylindole, 6-hydroxy-7-methoxy-2-ethoxycarbonylindole, 6-hydroxy-7-dimethylaminomethylindole, 6-hydroxy-3,7-dimethylindole, 6-hydroxy-3-methyl-7-dimethylaminomethylindole, 6-hydroxy-5-methoxy-1-methylindole, 6-hydroxy-5-methyl-2-carboxyindole, 6-hydroxy-7-methoxy-2-methylindole, 6-hydroxy-5-methoxy-2-methylindole and 6-hydroxy-5-acetylamino-2,3-dimethylindole.

3. Method according to either of Claims 1 or 2, characterized in that the para oxidation dye precursors are chosen from para-phenylenediamines, para-aminophenols, para heterocyclic precursors and double bases.

4. Method according to anyone of Claims 1 to 3, characterized in that the para-phenylenediamines are chosen from the compounds corresponding to the formula (II):

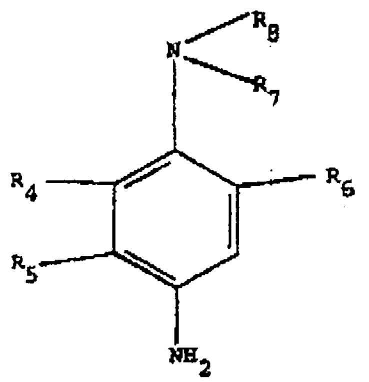

(II)

in which $R_4$, $R_5$ and $R_6$, which may be identical or different, represent a hydrogen or halogen atom, an alkyl radical having 1 to 4 carbon atoms or an alkoxy radical having 1 to 4 carbon atoms, and $R_7$ and $R_8$, which may be identical or different, represent a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl or morpholinoalkyl, in which the alkyl or alkoxy groups have from 1 to 4 carbon atoms, or $R_7$ and $R_8$ form, together with the nitrogen atom to which they are bonded, a piperidino or morpholino heterocycle, with the proviso that $R_4$ or $R_6$ represents a hydrogen atom when $R_7$ and $R_8$ do not represent a hydrogen atom, as well as the salts of these compounds.

5. Method according to Claim 4, characterized in that the compounds of formula (II) are chosen from p-phenylenediamine, p-tolylenediamine, methoxy-para-phenylenediamine, chloro-para-phenylenediamine, 2,6-dimethyl-para-phenylenediamine, 2,5-dimethyl-para-phenylenediamine, 2-methyl-5-methoxy-para-phenylenediamine, 2,6-dimethyl-5-methoxy-para-phenylenediamine, N,N'-dimethyl-para-phenylenedimaine, 3-methyl-4-amino-N,N-diethylaniline, N,N-di(β-hydroxyethyl)-para-phenylenediamine, 3-methyl-4-amino-N,N-di(β-hydroxyethyl)aniline, 3-chloro-4-amino-N,N-di(β-hydroxyethyl)aniline, 4-amino-N,N-(ethyl,carbamylmethyl)aniline, 3-methyl-4-amino-N,N-(ethyl,carbamylmethyl)aniline, 4-amino-N,N-(ethyl,β-piperidinoethyl)aniline, 3 methyl-4-amino-N,N-(ethyl,β-piperidinoethyl)aniline, 4-amino-N,N-(ethyl,β-morpholinoethyl)aniline, 3-methyl-4-amino-N,N-(ethyl,β-morpholinoethyl)aniline, 4-amino-N,N-(ethyl,β-acetylaminoethyl)aniline, 4-amino-N-(β-methoxyethyl)aniline, 3-methyl-4-amino-N,N-(ethyl,β-acetylaminoethyl)aniline, 4-amino-N,N-(ethyl,β-mesylaminoethyl)aniline, 3-methyl-4-amino-N,N-(ethyl,β-mesylaminoethyl)aniline, 4-amino-N,N-(ethyl,β-sulphoethyl)aniline, 3-methyl-4-amino-N,N-(ethyl,β-sulphoethyl)aniline, N-[(4'-amino)phenyl]morpholine and N-[(4'-amino)phenyl]piperidine, and their salts.

6. Method according to any one of Claims 1 to 4, characterized in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2-methoxymethyl-4-aminophenol and 2,5-dimethyl-4-aminophenol.

7. Method according to one of Claims 1 and 2, characterized in that the oxidation dye precursors of ortho type are chosen from ortho-aminophenols and ortho-phenylenediamines.

8. Method according to Claim 3, characterized in that the so-called double bases are bisphenylalklenediamines corresponding to the formula:

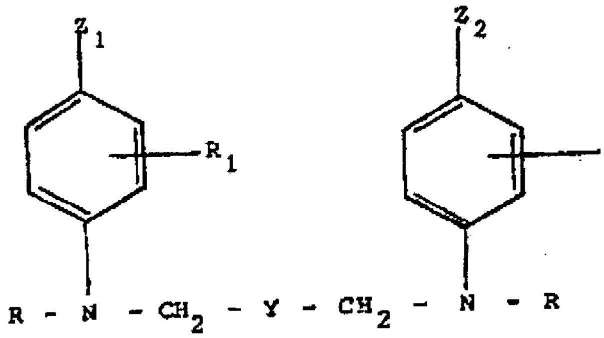

(III)

in which:

$Z_1$ and $Z_2$, which may be identical or different, represent hydroxyl or $NHR_3$ groups, where $R_3$ denotes a hydrogen atom or a lower alkyl radical;

$R_1$ and $R_2$, which may be identical or different, represent either hydrogen atoms or halogen atoms or alkyl groups;

R represents a hydrogen atom or an alkyl, hydroxyalkyl or aminoalkyl group, in which the amino radical may be substituted; and

Y represents a radical taken from the group comprising the following radicals: $-(CH_2)_n-$, $(CH_2)_{n'}$ -O-$(CH_2)_{n'}$-, $-(CH_2)_{n'}$-CHOH-$(CH_2)_{n'}$-$(CH_2)_{n'}$

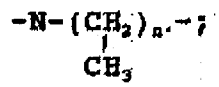

n being an integer between 0 and 8 and n' being an integer between 0 and 4, it being possible for this base to be in the form of its addition salts with acids.

**9.** Method according to Claim 8, characterized in that the compounds of formula (III) are chosen from N,N'-bis(β-hydroxyethyl)-N,N'-bis(4'-aminophenyl)-1,3-diamino-2-propanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxymethyl)-N,N'-bis(4-aminophenyl)tetramethylenediamine, N,N'-bis(4-methylaminophenyl)-tetramethylenediamine and N,N'-bis(ethyl)-N,N'-bis(4'-amino-3'-methylphenyl)ethylenediamine.

**10.** Method according to any one of Claims 1 to 9, characterized in that, in addition to the heterocyolic couplers of formula (I), meta-diphenols, meta-aminophenols, meta-phenylenediamines, meta-acylaminophenols, meta-ureidophenols, meta-carbalkoxyaminophenols, α-naphthol, β-ketone compounds, pyrazolones or 4-hydroxyindole are also used.

**11.** Method according to any one of Claims 1 to 10, characterized in that direct dyes are also used.

**12.** Tinctorial composition for keratinous fibres, characterized in that it contains, in a medium appropriate for dyeing these fibres, at least one compound corresponding to the formula:

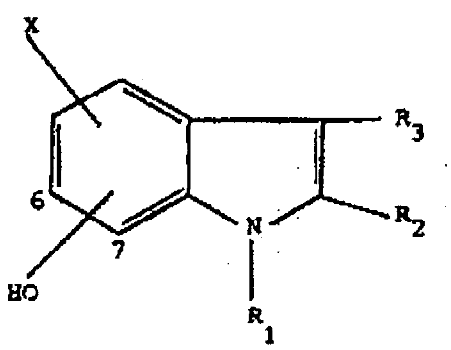

(I)

in which $R_1$ denotes a hydrogen atom or a $C_1$-$C_4$ alkyl radical; $R_2$ and $R_3$, which may be identical or different, denote a hydrogen atom, a $C_1$-$C_4$ lower alkyl radical, a carboxyl radical or an alkoxycarbonyl radical; X denotes a $C_1$-$C_4$ lower alkyl radical, a $C_1$-$C_{18}$ alkoxy radical, a halogen atom, a $C_1$-$C_{20}$ acyloxy radical, an acetylamino group, a trimethylsilyloxy group or a dialkyl($C_1$-$C_4$)-aminomethyl group; the group OH oocupying the 6- or 7-positions, as well as their salts as coupler(s) and at least one para and/or ortho oxidation dye precursor chosen from para-aminophenols, heterocyclic precursors, ortho-aminophenols, ortho-phenylenediamines, the so-called double bases of the family of bisphenylalklenediamines, and para-phenylenediamines of formula:

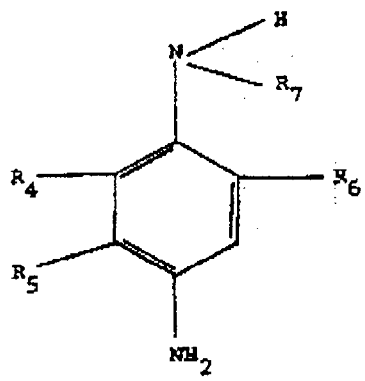

(IV)

in which $R_4$, $R_5$ and $R_6$, which may be identical or different, represent a hydrogen or halogen atom, an alkyl radical having 1 to 4 carbon atoms or an alkoxy radical having 1 to 4 carbon atoms, and $R_7$ represents a hydrogen atom or an alkyl, carbamylalkyl, mesylaminoalkyl, acetylaminoalkyl, ureidoalkyl, carbalkoxyaminoalkyl, piperidinoalkyl or morpholinoalkyl radical, these alkyl or alkoxy groups having from 1 to 4 carbon atoms, as well as the salts of those compounds, this composition containing no iodide ion.

**13.** Composition according to Claim 12, characterized in that the para-phenylenediamines are chosen from p-phenylenediamine, p-tolylenediamine, methoxy-para-phenylenediamine, chloro-para-phenylenediamine, 2,6-dimethyl-para-phenylenediamine, 2,5-dimethyl-para-phenylenediamine, 2-methyl-5-methoxy-para-phenylenediamine and 2,6-dimethyl-5-methoxy-para-phenylenediamine.

**14.** Composition according to either of Claims 12 and 13, characterized in that the oxidation dyes of para and/or ortho type and the couplers are present in proportions of between 0.3 and 7% by weight relative to the total weight of the composition.

**15.** Composition according to any one of Claims 12 to 14, characterized in that the compound of formula (I) is present in proportions of between 0.05 and 3.5% by weight relative to the total weight of the composition.

**16.** Composition according to any one of Claims 12 to 15, characterized in that the medium appropriate for dyeing these fibres is aqueous and in that it has a pH of between 8 and 11.

**17.** Composition according to any one of Claims 12 to 16, charactarized in that the composition also contains anionic, cationic, nonionic or amphoteric surface-active agents or their mixtures.

**18.** Composition according to any one of Claims 12 to 17, characterized in that the composition also contains organic solvents in proportions of between 1 and 40% by weight relative to the total weight of the composition.

**19.** Composition according to many one of Claims 12 to 18, characterized in that the composition also contains thickeners, antioxidants, penetrating agents, sequestering agents, buffers and/or perfumes.

**20.** Composition according to any one of Claims 12 to 19, characterized in that it is in the form of liquids, creams or gels and in that it is optionally packaged as aerosols in the presence of a propellant.

**21.** Method for dyeing keratinous fibres, in particular human hair, according to one of Claims 1 to 11, characterized in that at least one composition as defined in one of Claims 12 to 20 and at least one oxidizing agent containing hydrogen peroxide are applied thereto, the application lasting 10 to 40 minutes and being followed by rinsing, if appropriate washing and drying.

**22.** Method of preparation of 6-hydroxy-5-acetylamino-2,3-dimethylindole, characterized in that 5-amino-2,3-dimethyl-6-hydroxyindole is mixed with sodium sulphite, acetic anhydride is added, and the title compound is recovered.

**23.** Method of preparation of a composition intended to be used for dyeing keratinous fibres, characterized by introducing, into a medium appropriate for dyeing, at least one compound corresponding to the formula:

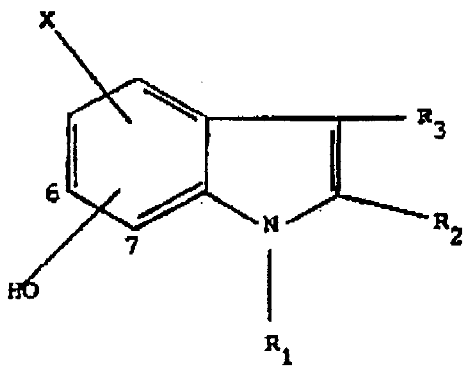

(I)

## Patentansprüche

## Patentansprüche für folgende Vertragsstaaten : AT, BE, CH, DE, DK, FR, GB, GR, IT, LI, NL, SE

**1.** Verfahren zur Färbung keratinischer Fasern, insbesondere menschlicher Haare, dadurch **gekennzeichnet**, daß man auf diese eine Zusammensetzung aufträgt, die mindestens eine Verbindung der Formel:

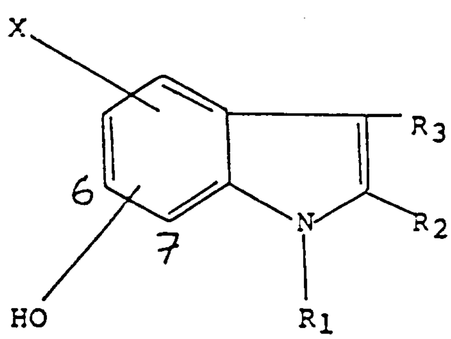

(I)

worin $R_1$ ein Wasserstoffatom oder einen $C_{1-4}$-Alkylrest, $R_2$ und $R_3$, gleich oder verschieden, ein Wasserstoffatom, einen $C_{1-4}$-Niedrigalkyl-, Carboxyl- oder Alkoxycarbonylrest und X einen $C_{1-4}$-Niedrigalkylrest, einen $C_{1-18}$-Alkoxyrest, ein Halogenatom, einen $C_{1-20}$-Acyloxyrest, eine Acetylamino-, Trimethylsilyloxy- oder $C_{1-4}$-Dialkylaminomethylgruppe darstellen, wobei die OH-Gruppe die Position 6 oder 7 des aromatischen Rings einnimmt, sowie deren Salze, als Kuppler,
und mindestens eine Oxidationsfarbstoff-Vorstufe vom p- und/oder o-Typ enthält,
und daß man auf die Fasern ein oxidierendes Mittel aufbringt, das Wasserstoffperoxid enthält, wobei das oxidierende Mittel entweder kurz vor der Anwendung in die die genannte Verbindung der Formel (I) und die Oxidationsfarbstoff-Vorstufe enthaltende Zusammensetzung eingebracht oder auf die Fasern gegeben wird, und zwar ohne Anwendung von Jodidion.

2. Verfahren gemäß Anspruch 1, dadurch **gekennzeichnet**, daß die Verbindungen der Formel (I) aus 6-Hydroxy-5-acetoxyindol, 6-Hydroxy-5-methoxyindol, 6-Hydroxy-5-methoxy-2-methylindol, 6-Hydroxy-5-butoxyindol, 6-Hydroxy-5-methoxyindol-2-carboxylsäure, 6-Hydroxy-7-methoxyindol, 6-Hydroxy-5-methoxy-2,3-dimethylindol, 7-Hydroxy-4-methoxy-2,3-dimethylindol, 6-Hydroxy-5-tetradecanoyloxyindol, 6-Hydroxy-5-hexadecyloxyindol, 6-Hydroxy-5-butanoyloxyindol, 6-Hydroxy-5-oleyloxyindol, 6-Hydroxy-5-ethanoyloxyindol, 6-Hydroxy-5-hexanoyloxyindol, 6-Hydroxy-5-pivaloyloxyindol, 6-Hydroxy-5-formyloxyindol, 6-Hydroxy-5-trimethylsilyloxyindol, 6-Hydroxy-7-methylindol, 7-Hydroxy-6-dimethylaminomethylindol, 7-Hydroxy-6-methoxy-2-methylindol, 6-Hydroxy-7-methoxy-2-ethoxycarbonylindol, 6-Hydroxy-7-dimethylaminomethylindol, 6-Hydroxy-3,7-dimethylindol, 6-Hydroxy-3-methyl-7-dimethylaminomethylindol, 6-Hydroxy-5-methoxy-1-methylindol, 6-Hydroxy-5-methyl-2-carboxylindol, 6-Hydroxy-7-methoxy-2-methylindol, 6-Hydroxy-5-methoxy-2-methylindol, 6-Hydroxy-5-acetylamino-2,3-dimethylindol ausgewählt sind.

3. Verfahren gemäß jedem Anspruch 1 oder 2, dadurch **gekennzeichnet**, daß die Oxidationsfarbstoff-Vorstufen vom para-Typ aus p-Phenylendiaminen, p-Aminophenolen, heterozycklischen Vorstufen-Verbindungen vom para-Typ und aus Doppelbasen ausgewählt sind.

4. Verfahren gemäß jedem Anspruch 1 bis 3, dadurch **gekennzeichnet**, daß die p-Phenylendiamine aus Verbindungen der Formel (II):

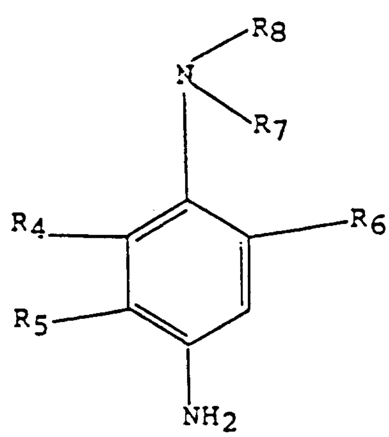

(II)

worin $R_4$, $R_5$ und $R_6$ , gleich oder verschieden, ein Wasserstoff- oder Halogenatom, einen Alkylrest mit 1 bis 4 Kohlenstoffatomen, einen Alkoxyrest mit 1 bis 4 Kohlenstoffatomen und $R_7$ und $R_8$, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Piperidinoalkyl- oder Morpholinoalkylrest darstellen, wobei diese Alkyl- oder Alkoxygruppen 1 bis 4 Kohlenstoffatome aufweisen, oder worin $R_7$ und $R_8$ zusammen mit dem Stickstoffatom, an das sie gebunden sind, auch einen Piperidin- oder Morpholinheterozyklus bilden, mit der Maßgabe, daß $R_4$ oder $R_6$ ein Wasserstoffatom darstellen, wenn $R_7$ und $R_8$ kein Wasserstoffatom darstellen, sowie aus den Salzen dieser Verbindungen ausgewählt sind.

5. Verfahren gemäß Anspruch 4, dadurch **gekennzeichnet**, daß die Verbindungen der Formel (II) aus p-Phenylendiamin, p-Toluylendiamin, Methoxy-p-phenylendiamin, Chlor-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 4-Amino-N,N-(ethyl,carbamylmethyl)anilin, 3-Methyl-4-amino-N,N-(ethyl,carbamylmethyl)anilin, 4-Amino-N,N-(ethyl,β-piperidinoethyl)anilin, 3-Methyl-4-amino-N,N-(ethyl,β-piperidinoethyl)anilin, 4-Amino-N,N-(ethyl,β-morpholinoethyl)anilin, 3-Methyl-4-amino-N,N-(ethyl,β-morpholinoethyl)anilin, 4-Amino-N,N-(ethyl,β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl, β-mesylaminoethyl)anilin, 4-Amino-N,N-(ethyl-β-sulfoethyl)anilin, 3-Methyl-4-amino-N,N-(ethyl,β-sulfoethyl)anilin, N-((4'-Amino)-phenyl)morpholin, N-((4'-Amino)phenyl)piperidin und aus deren Salzen ausgewählt sind.

6. Verfahren gemäß jedem Anspruch 1 bis 4,
dadurch **gekennzeichnet**, daß
die p-Aminophenole aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol ausgewählt sind.

7. Verfahren gemäß einem der Ansprüche 1 und 2,
dadurch **gekennzeichnet**, daß
die Oxidationsfarbstoff-Vorstufen vom ortho-Typ aus o-Aminophenolen und o-Phenylendiaminen augewählt sind.

8. Verfahren gemäß Anspruch 3,
das die als doppelte bezeichnete Basen Bisphenylalkylendiamine der Formel sind:

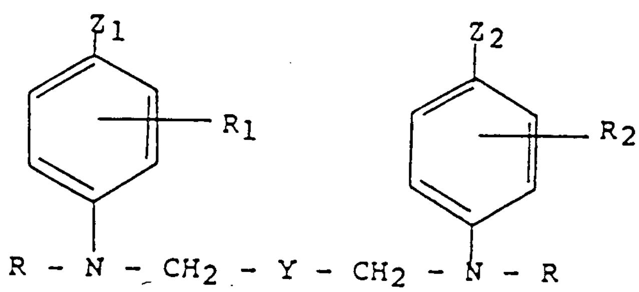

(III)

worin gilt:
$Z_1$ und $Z_2$, gleich oder verschieden, stellen Hydroxyl- oder $NHR_3$-Gruppen dar, worin $R_3$ ein Wasserstoffatom oder einen Niedrigalkylrest bedeutet:
$R_1$ und $R_2$, gleich oder verschieden, stellen entweder Wasserstoffatome oder Halogenatome oder auch Alkylgruppen dar; R stellt ein Wasserstoffatom, eine Alkyl-, Hydroxyalkyl- oder Aminoalkylgruppe dar, worin der Aminorest substituiert sein kann;
Y stellt einen Rest aus der durch die folgenden Reste dargestellten Gruppe dar: $-(CH_2)_n-$, $(CH_2)_{n'}-O-(CH_2)_{n'}-$, $-(CH_2)_{n'}-CHOH-(CH_2)_{n'}-$,
$-(CH_2)_n-N-CH_3-(CH_2)_{n'}-$,
worin n eine ganze Zahl von 0 bis 8 und n' eine ganze Zahl von 0 bis 4 sind, wobei diese Base in Form ihrer Additionssalze mit Säuren vorliegen kann.

9. Verfahren gemäß Anspruch 8,
dadurch **gekennzeichnet**, daß
die Verbindungen der Formel (III) aus N,N'-Bis(β-hydroxyethyl)N,N'-bis(4'-aminophenyl)-1,3-diamino-2-propanol, N,N'-Bis(β-hydroxyethyl)N,N'-bis(4'-aminophenyl)ethylendiamin, N,N'-Bis(4-aminophenyl)-tetramethylendiamin, N,N'-Bis(β-hydroxyethyl)N,N'-bis(4-aminophenyl)tetramethylendiamin, N,N'-(Bis(4-methylaminophenyl)tetramethylendiamin, N,N'-Bis(ethyl)N,N'-bis(4'-amino-3'-methylphenyl)-ethylendiamin ausgewählt sind.

10. Verfahren gemäß jedem Anspruch 1 bis 9,
dadurch **gekennzeichnet**, daß
man auch zusätzlich zu den heterozyklischen Kupplern der Formel (I) m-Diphenole, m-Aminophenole, m-Phenylendiamine; m-Acylaminophenole, m-Ureidophenole, m-Carbalkoxyaminophenole, α -Naphthol,

*β*-Ketoverbindungen, Pyrazolone oder 4-Hydroxyindol zur Anwendung bringt.

**11.** Verfahren gemäß jedem Anspruch 1 bis 10, dadurch **gekennzeichnet**, daß man auch Direktfarbstoffe verwendet.

**12.** Färbezusammensetzung für keratinische Fasern, dadurch **gekennzeichnet**, daß sie in einem zur Färbung dieser Fasern geeigneten Milieu mindestens eine Verbindung der Formel:

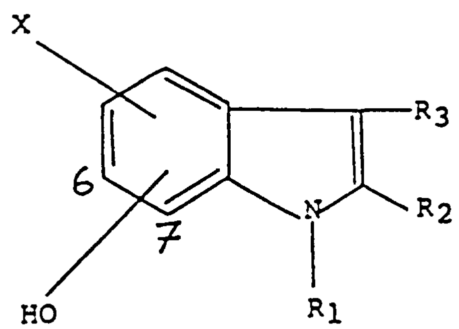

(I)

worin $R_1$ ein Wasserstoffatom oder einen $C_{1-4}$-Alkylrest, $R_2$ und $R_3$, gleich oder verschieden, ein Wasserstoffatom, einen $C_{1-4}$-Niedrigalkyl-, Carboxyl- oder Alkoxycarbonylrest und X einen $C_{1-4}$-Niedrigalkylrest, einen $C_{1-18}$-Alkoxyrest, ein Halogenatom, einen $C_{1-20}$-Acyloxyrest, eine Acetylamino-, Trimethylsilyloxy- oder $C_1$-$C_4$-Dialkylaminomethylgruppe darstellen, wobei die OH-Gruppe die Position 6 oder 7 des aromatischen Rings einnimmt, sowie deren Salze, als Kuppler und mindestens eine Oxidationsfarbstoff-Vorstufe vom para- und/oder ortho-Typ enthält, ausgewählt aus p-Aminophenolen, heterozyklischen Vorstufen-Verbindungen, o-Aminophenolen, o-Phenylendiaminen, als Doppelbasen bezeichneten Basen der Familie der Bisphenylalkylendiamine, p-Phenylendiaminen der Formel:

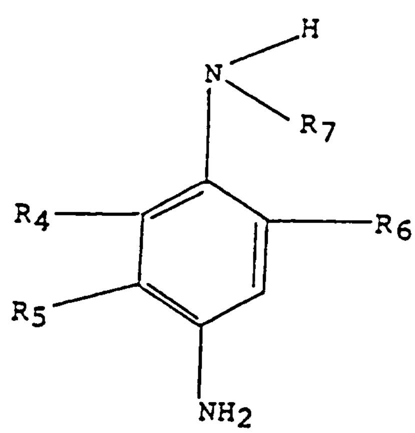

(IV)

worin $R_4$, $R_5$ und $R_6$, gleich oder verschieden, ein Wasserstoff- oder Halogenatom, einen Alkylrest mit 1 bis 4 Kohlenstoffatomen oder einen Alkoxyrest mit 1 bis 4 Kohlenstoffatomen und $R_7$ ein Wasserstoffatom, einen Alkyl-, Carbamylalkyl-, Mesylaminoalkyl-, Acetylaminoalkyl-, Ureidoalkyl-, Carbalkoxyaminoalkyl-, Piperidinoalkyl- oder Morpholinoalkylrest darstellen, wobei diese Alkyl- oder Alkoxygruppen 1 bis 4 Kohlenstoffatome aufweisen, sowie ausgewählt aus den Salzen dieser Verbindungen, wobei diese Zusammensetzung kein Jodidion enthält.

**13.** Zusammensetzung gemäß Anspruch 12, dadurch **gekennzeichnet**, daß die p-Phenylendiamine aus p-Phenylendiamin p-Toluylendiamin, Methoxy-p-phenylendiamin, Chlor-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin ausgewählt sind.

**14.** Zusammensetzung gemäß jedem Anspruch 12 und 13,
dadurch **gekennzeichnet**, daß
die Oxidationsfarbstoffe vom para- und/oder ortho-Typ und die Kuppler in Anteilen von 0,3 bis 7 Gew.%, bezogen auf das Gesamtgewicht der Zusammensetzung, vorhanden sind.

**15.** Zusammensetzung gemäß jedem Anspruch 12 bis 14,
dadurch **gekennzeichnet**, daß
die Verbindung der Formel (I) in Mengenanteilen von 0,05 bis 3,5 Gew.%, bezogen auf das Gesamtgewicht der Zusammensetzung, vorliegt.

**16.** Zusammensetzung gemäß jedem Anspruch 12 bis 15,
dadurch **gekennzeichnet**, daß
das zur Färbung der Fasern geeignete Milieu wässrig ist und einen pH von 8 bis 11 aufweist.

**17.** Zusammensetzung gemäß jedem Anspruch 12 bis 16,
dadurch **gekennzeichnet**, daß
die Zusammensetzung auch anionische, kationische, nichtionische oder amphotere oberflächenaktive Mittel oder deren Mischungen enthält.

**18.** Zusammensetzung gemäß jedem Anspruch 12 bis 17,
dadurch **gekennzeichnet**, daß
die Zusammensetzung auch organische Lösungsmittel in Mengenanteilen von 1 bis 40 Gew.%, bezogen auf das Gesamtgewicht der Zusammensetzung, enthält.

**19.** Zusammensetzung gemäß jedem Anspruch 12 bis 18,
dadurch **gekennzeichnet**, daß
die Zusammensetzung auch Verdickungsmittel, Antioxidantien, Eindringmittel, Sequestriermittel, Puffer und/oder Parfüm-Produkte enthält.

**20.** Zusammensetzung gemäß jedem Anspruch 12 bis 19,
dadurch **gekennzeichnet**, daß
sie in Form von Flüssigkeiten, Creme-Produkten, Gelen vorliegt und gegebenenfalls als Aerosol in Gegenwart eines Treibmittels zubereitet ist.

**21.** Verfahren zur Färbung keratinischer Fasern, insbesondere menschlicher Haare, gemäß einem der Ansprüche 1 bis 11,
dadurch **gekennzeichnet**, daß
man auf diese mindestens eine in einem der Ansprüche 12 bis 20 definierte Zusammensetzung und mindestens ein oxidierendes Mittel aufträgt, das Wasserstoffperoxid enthält, wobei die Anwendung 10 bis 40 Minuten lang dauert und im Anschluß daran gespült, gegebenenfalls gewaschen und getrocknet wird.

**22.** Neue Verbindung, dargestellt durch 6-Hydroxy-5-acetylamino-2,3-dimethylindol.

**Patentansprüche für folgenden Vertragsstaat : ES**

**1.** Verfahren zur Färbung keratinischer Fasern, insbesondere menschlicher Haare,
dadurch **gekennzeichnet**, daß
man auf diese eine Zusammensetzung aufträgt, die mindestens eine Verbindung der Formel:

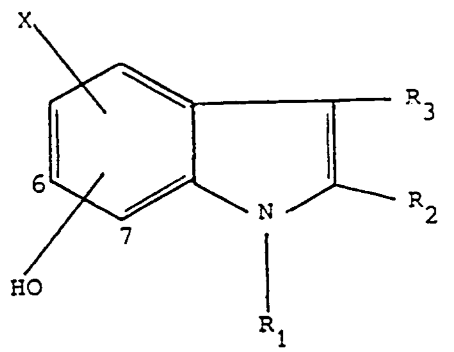

(I)

worin $R_1$ ein Wasserstoffatom oder einen $C_{1-4}$-Alkylrest, $R_2$ und $R_3$, gleich oder verschieden, ein Wasserstoffatom, einen $C_{1-4}$-Niedrigalkyl-, Carboxyl- oder Alkoxycarbonylrest und X einen $C_{1-4}$-Niedrigalkylrest, einen $C_{1-18}$-Alkoxyrest, ein Halogenatom, einen $C_{1-20}$-Acyloxyrest, eine Acetylamino-, Trimethylsilyloxy- oder $C_{1-4}$-Dialkylaminomethylgruppe darstellen, wobei die OH-Gruppe die Position 6 oder 7 des aromatischen Rings einnimmt, sowie deren Salze, als Kuppler,
und mindestens eine Oxidationsfarbstoff-Vorstufe vom p- und/oder o-Typ enthält,
und daß man auf die Fasern ein oxidierendes Mittel aufbringt, das Wasserstoffperoxid enthält, wobei das oxidierende Mittel entweder kurz vor der Anwendung in die die genannte Verbindung der Formel (I) und die Oxidationsfarbstoff-Vorstufe enthaltende Zusammensetzung eingebracht oder auf die Fasern gegeben wird, und zwar ohne Anwendung von Jodidion.

**2.** Verfahren gemäß Anspruch 1,
dadurch **gekennzeichnet**, daß
die Verbindungen der Formel (I) aus 6-Hydroxy-5-acetoxyindol, 6-Hydroxy-5-methoxyindol, 6-Hydroxy-5-methoxy-2-methylindol, 6-Hydroxy-5-butoxyindol, 6-Hydroxy-5-methoxyindol-2-carboxylsäure, 6-Hydroxy-7-methoxyindol, 6-Hydroxy-5-methoxy-2,3-dimethylindol, 7-Hydroxy-4-methoxy-2,3-dimethylindol, 6-Hydroxy-5-tetradecanoyloxyindol, 6-Hydroxy-5-hexadecyloxyindol, 6-Hydroxy-5-butanoyloxyindol, 6-Hydroxy-5-oleyloxyindol, 6-Hydroxy-5-ethanoyloxyindol, 6-Hydroxy-5-hexanoyloxyindol, 6-Hydroxy-5-pivaloyloxyindol, 6-Hydroxy-5-formyloxyindol, 6-Hydroxy-5-trimethylsilyloxyindol, 6-Hydroxy-7-methylindol, 7-Hydroxy-6-dimethylaminomethylindol, 7-Hydroxy-6-methoxy-2-methylindol, 6-Hydroxy-7-methoxy-2-ethoxycarbonylindol, 6-Hydroxy-7-dimethylaminomethylindol, 6-Hydroxy-3,7-dimethylindol, 6-Hydroxy-3-methyl-7-dimethylaminomethylindol, 6-Hydroxy-5-methoxy-1-methylindol, 6-Hydroxy-5-methyl-2-carboxylindol, 6-Hydroxy-7-methoxy-2-methylindol, 6-Hydroxy-5-methoxy-2-methylindol, 6-Hydroxy-5-acetylamino-2,3-dimethylindol ausgewählt sind.

**3.** Verfahren gemäß jedem Anspruch 1 oder 2,
dadurch **gekennzeichnet**, daß
die Oxidationsfarbstoff-Vorstufen vom para-Typ aus p-Phenylendiaminen, p-Aminophenolen, heterozycklischen Vorstufen-Verbindungen vom para-Typ und aus Doppelbasen ausgewählt sind.

**4.** Verfahren gemäß jedem Anspruch 1 bis 3,
dadurch **gekennzeichnet**, daß
die p-Phenylendiamine aus Verbindungen der Formel (II):

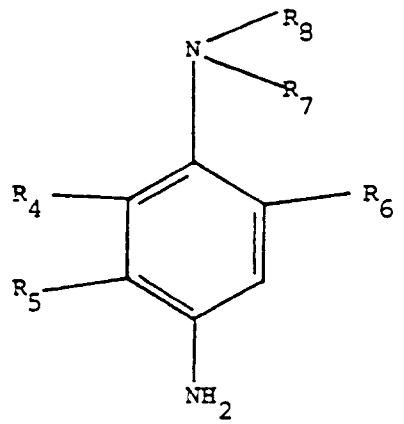

(II)

worin $R_4$, $R_5$ und $R_6$ , gleich oder verschieden, ein Wasserstoff- oder Halogenatom, einen Alkylrest mit 1 bis 4 Kohlenstoffatomen, einen Alkoxyrest mit 1 bis 4 Kohlenstoffatomen und $R_7$ und $R_8$, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Piperidinoalkyl- oder Morpholinoalkylrest darstellen, wobei diese Alkyl- oder Alkoxygruppen 1 bis 4 Kohlenstoffatome aufweisen, oder worin $R_7$ und $R_8$ zusammen mit dem Stickstoffatom, an das sie gebunden sind, auch einen Piperidin- oder Morpholinheterozyklus bilden, mit der Maßgabe, daß $R_4$ oder $R_6$ ein Wasserstoffatom darstellen, wenn $R_7$ und $R_8$ kein Wasserstoffatom darstellen, sowie aus den Salzen dieser Verbindungen ausgewählt sind.

**5.** Verfahren gemäß Anspruch 4,
dadurch **gekennzeichnet**, daß
die Verbindungen der Formel (II) aus p-Phenylendiamin, p-Toluylendiamin, Methoxy-p-phenylendiamin, Chlor-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 4-Amino-N,N-(ethyl,carbamylmethyl)anilin, 3-Methyl-4-amino-N,N(ethyl,carbamylmethyl)anilin, 4-Amino-N,N-(ethyl,β-piperidinoethyl)anilin, 3-Methyl-4-amino-N,N-(ethyl,β-piperidinoethyl)anilin, 4-Amino-N,N-(ethyl,β-morpholinoethyl)anilin, 3-Methyl-4-amino-N,N-(ethyl,β-morpholinoethyl)anilin, 4-Amino-N,N-(ethyl,β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl-β-mesylaminoethyl)anilin, 4-Amino-N,N-(ethyl, β-sulfoethyl)anilin, 3-Methyl-4-amino-N,N-(ethyl,β-sulfoethyl)anilin, N-((4'-Amino)-phenyl)morpholin, N-((4'-Amino)phenyl)piperidin und aus deren Salzen ausgewählt sind.

**6.** Verfahren gemäß jedem Anspruch 1 bis 4,
dadurch **gekennzeichnet**, daß
die p-Aminophenole aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol ausgewählt sind.

**7.** Verfahren gemäß einem der Ansprüche 1 und 2,
dadurch **gekennzeichnet**, daß
die Oxidationsfarbstoff-Vorstufen vom ortho-Typ aus o-Aminophenolen und o-Phenylendiaminen augewählt sind.

**8.** Verfahren gemäß Anspruch 3,
das die als doppelte bezeichnete Basen Bisphenylalkylendiamine der Formel sind:

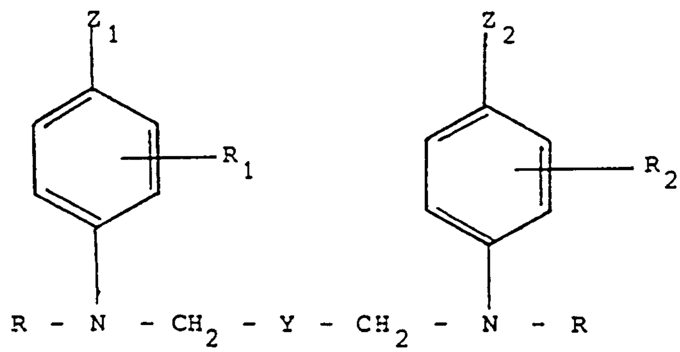

(III)

worin gilt:
$Z_1$ und $Z_2$, gleich oder verschieden, stellen Hydroxyl- oder $NHR_3$-Gruppen dar, worin $R_3$ ein Wasserstoffatom oder einen Niedrigalkylrest bedeutet:
$R_1$ und $R_2$, gleich oder verschieden, stellen entweder Wasserstoffatome oder Halogenatome oder auch Alkylgruppen dar; R stellt ein Wasserstoffatom, eine Alkyl-, Hydroxyalkyl- oder Aminoalkylgruppe dar, worin der Aminorest substituiert sein kann;
Y stellt einen Rest aus der durch die folgenden Reste dargestellten Gruppe dar: $-(CH_2)_n-$, $(CH_2)_{n'}-O-(CH_2)_{n'}-$, $-(CH_2)_{n'}-CHOH-(CH_2)_{n'}-$,
$-(CH_2)_{n'}-N-CH_3-(CH_2)_{n'}-$,
worin n eine ganze Zahl von 0 bis 8 und n' eine ganze Zahl von 0 bis 4 sind, wobei diese Base in Form ihrer Additionssalze mit Säuren vorliegen kann.

**9.** Verfahren gemäß Anspruch 8,
dadurch **gekennzeichnet**, daß
die Verbindungen der Formel (III) aus N,N'-Bis(β-hydroxyethyl)N,N'-bis(4'-aminophenyl)-1,3-diamino-2-propanol, N,N'-Bis(β-hydroxyethyl)N,N'-bis(4'-aminophenyl)ethylendiamin, N,N'-Bis(4-aminophenyl)-tetramethylendiamin, N,N'-Bis(β-hydroxyethyl)N,N'-bis(4-aminophenyl)tetramethylendiamin, N,N'-(Bis(4-methylaminophenyl)tetramethylendiamin, N,N'-Bis(ethyl)N,N'-bis(4'-amino-3'-methylphenyl)-ethylendiamin ausgewählt sind.

**10.** Verfahren gemäß jedem Anspruch 1 bis 9,
dadurch **gekennzeichnet**, daß
man auch zusätzlich zu den heterozyklischen Kupplern der Formel (I) m-Diphenole, m-Aminophenole, m-Phenylendiamine; m-Acylaminophenole, m-Ureidophenole, m-Carbalkoxyaminophenole, α -Naphthol, β-Ketoverbindungen, Pyrazolone oder 4-Hydroxyindol zur Anwendung bringt.

**11.** Verfahren gemäß jedem Anspruch 1 bis 10,
dadurch **gekennzeichnet**, daß
man auch Direktfarbstoffe verwendet.

**12.** Färbezusammensetzung für keratinische Fasern,
dadurch **gekennzeichnet**, daß,
sie in einem zur Färbung dieser Fasern geeigneten Milieu mindestens eine Verbindung der Formel:

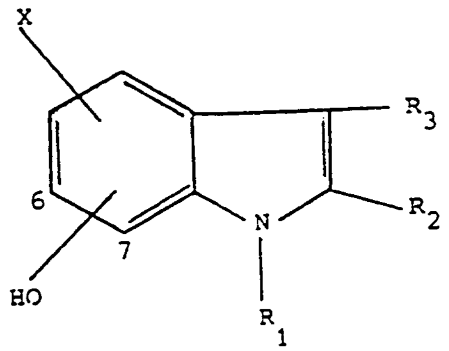

(I)

worin $R_1$ ein Wasserstoffatom oder einen $C_{1-4}$-Alkylrest, $R_2$ und $R_3$, gleich oder verschieden, ein Wasserstoffatom, einen $C_{1-4}$-Niedrigalkyl-, Carboxyl- oder Alkoxycarbonylrest und X einen $C_{1-4}$-Niedrigalkylrest, einen $C_{1-18}$-Alkoxyrest, ein Halogenatom, einen $C_{1-20}$-Acyloxyrest, eine Acetylamino-, Trimethylsilyloxy- oder $C_{1-4}$-Dialkylaminomethylgruppe darstellen, wobei die OH-Gruppe die Position 6 oder 7 des aromatischen Rings einnimmt, sowie deren Salze, als Kuppler und mindestens eine Oxidationsfarbstoff-Vorstufe vom para- und/oder ortho-Typ enthält, ausgewählt aus p-Aminophenolen, heterozyklischen Vorstufen-Verbindungen, o-Aminophenolen, o-Phenylendiaminen, als Doppelbasen bezeichneten Basen der Familie der Bisphenylalkylendiamine, p-Phenylendiaminen der Formel:

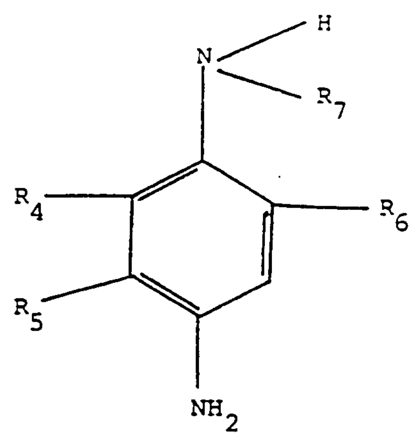

(IV)

worin $R_4$, $R_5$ und $R_6$, gleich oder verschieden, ein Wasserstoff- oder Halogenatom, einen Alkylrest mit 1 bis 4 Kohlenstoffatomen oder einen Alkoxyrest mit 1 bis 4 Kohlenstoffatomen und $R_7$ ein Wasserstoffatom, einen Alkyl-, Carbamylalkyl-, Mesylaminoalkyl-, Acetylaminoalkyl-, Ureidoalkyl-, Carbalkoxyaminoalkyl-, Piperidinoalkyl- oder Morpholinoalkylrest darstellen, wobei diese Alkyl- oder Alkoxygruppen 1 bis 4 Kohlenstoffatome aufweisen, sowie ausgewählt aus den Salzen dieser Verbindungen, wobei diese Zusammensetzung kein Jodidion enthält.

**13.** Zusammensetzung gemäß Anspruch 12,
dadurch **gekennzeichnet**, daß die p-Phenylendiamine aus
p-Phenylendiamin p-Toluylendiamin, Methoxy-p-phenylendiamin, Chlor-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin ausgewählt sind.

**14.** Zusammensetzung gemäß jedem Anspruch 12 und 13,
dadurch **gekennzeichnet**, daß
die Oxidationsfarbstoffe vom para- und/oder ortho-Typ und die Kuppler in Anteilen von 0,3 bis 7 Gew.%, bezogen auf das Gesamtgewicht der Zusammensetzung, vorhanden sind.

**15.** Zusammensetzung gemäß jedem Anspruch 12 bis 14,
dadurch **gekennzeichnet**, daß

die Verbindung der Formel (I) in Mengenanteilen von 0,05 bis 3,5 Gew.%, bezogen auf das Gesamtgewicht der Zusammensetzung, vorliegt.

**16.** Zusammensetzung gemäß jedem Anspruch 12 bis 15,
dadurch **gekennzeichnet**, daß
das zur Färbung der Fasern geeignete Milieu wässrig ist und einen pH von 8 bis 11 aufweist.

**17.** Zusammensetzung gemäß jedem Anspruch 12 bis 16,
dadurch **gekennzeichnet**, daß
die Zusammensetzung auch anionische, kationische, nichtionische oder amphotere oberflächenaktive Mittel oder deren Mischungen enthält.

**18.** Zusammensetzung gemäß jedem Anspruch 12 bis 17,
dadurch **gekennzeichnet**, daß
die Zusammensetzung auch organische Lösungsmittel in Mengenanteilen von 1 bis 40 Gew.%, bezogen auf das Gesamtgewicht der Zusammensetzung, enthält.

**19.** Zusammensetzung gemaß jedem Anspruch 12 bis 18,
dadurch **gekennzeichnet**, daß
die Zusammensetzung auch Verdickungsmittel, Antioxidantien, Eindringmittel, Sequestriermittel, Puffer und/oder Parfüm-Produkte enthält.

**20.** Zusammensetzung gemäß jedem Anspruch 12 bis 19,
dadurch **gekennzeichnet**, daß
sie in Form von Flüssigkeiten, Creme-Produkten, Gelen vorliegt und gegebenenfalls als Aerosol in Gegenwart eines Treibmittels zubereitet ist.

**21.** Verfahren zur Färbung keratinischer Fasern, insbesondere menschlicher Haare, gemäß einem der Ansprüche 1 bis 11,
dadurch **gekennzeichnet**, daß
man auf diese mindestens eine in einem der Ansprüche 12 bis 20 definierte Zusammensetzung und mindestens ein oxidierendes Mittel aufträgt, das Wasserstoffperoxid enthält, wobei die Anwendung 10 bis 40 Minuten lang dauert und im Anschluß daran gespült, gegebenenfalls gewaschen und getrocknet wird.

**22.** Verfahren zur Herstellung des 6-Hydroxy-5-acetylamino-2,3-dimethylindols,
dadurch **gekennzeichnet**, daß
man 5-Amino-2,3-dimethyl-6-hydroxyindol mit Natriumsulfit vermischt, Essigsäureanhydrid zufügt und die Titelverbindung gewinnt.

**23.** Verfahren zur Herstellung einer Zusammensetzung zur Verwendung zur Färbung keratinischer Fasern,
dadurch **gekennzeichnet**, daß
man in ein zur Färbung geeignetes Milieu mindestens eine Verbindung der Formel:

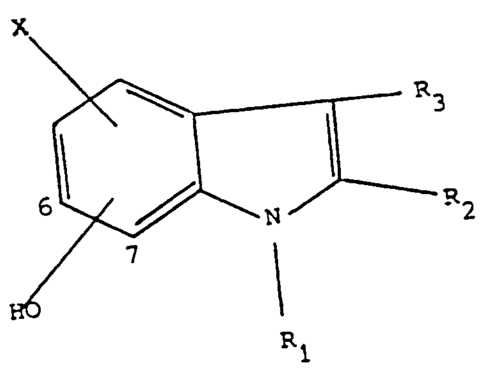

(I)

worin $R_1$ ein Wasserstoffatom oder einen $C_{1-4}$-Alkylrest, $R_2$ und $R_3$, gleich oder verschieden, ein

Wasserstoffatom, einen $C_{1-4}$-Niedrigalkyl-, Carboxyl- oder Alkoxycarbonylrest und X einen $C_{1-4}$-Niedrigalkylrest, einen $C_{1-18}$-Alkoxyrest, ein Halogenatom, einen $C_{1-20}$-Acyloxyrest, eine Acetylamino-, Trimethylsilyloxy- oder $C_{1-4}$-Dialkylaminomethylgruppe darstellen, wobei die OH-Gruppe die Position 6 oder 7 des aromatischen Rings einnimmt, sowie deren Salze, als Kuppler und mindestens eine Oxidationsfarbstoff-Vorstufe vom para- und/oder ortho-Typ, ausgewählt aus p-Aminophenolen, heterozyklischen Vorstufen-Verbindungen, o-Aminophenolen, o-Phenylendiaminen, als Doppelbasen bezeichneten Basen der Familie der Bisphenylalkylendiamine, p-Phenylendiaminen der Formel:

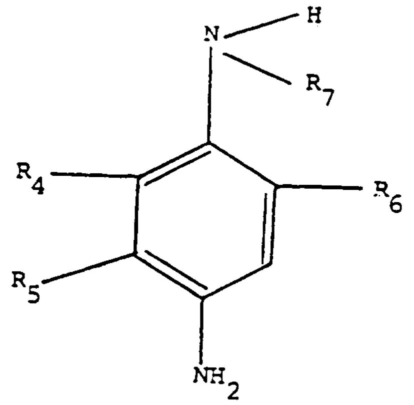

(IV)

worin $R_4$, $R_5$ und $R_6$, gleich oder verschieden, ein Wasserstoff- oder Halogenatom, einen Alkylrest mit 1 bis 4 Kohlenstoffatomen oder einen Alkoxyrest mit 1 bis 4 Kohlenstoffatomen und $R_7$ ein Wasserstoffatom, einen Alkyl-, Carbamylalkyl-, Mesylaminoalkyl-, Acetylaminoalkyl-, Ureidoalkyl-, Carbalkoxyaminoalkyl-, Piperidinoalkyl- oder Morpholinoalkylrest darstellen, wobei diese Alkyl- oder Alkoxygruppen 1 bis 4 Kohlenstoffatome aufweisen, sowie ausgewählt aus den Salzen dieser Verbindungen, in Mengenanteilen von 0,3 bis 7 Gew.%, bezogen auf das Gesamtgewicht der Zusammensetzung, einbringt.